# EUROPEAN PATENT SPECIFICATION

(11) **EP 2 704 636 B1**
(45) Date of publication and mention of the grant of the patent: **31.05.2017**
(21) Application number: 12779909.6
(22) Date of filing: 19.04.2012
(51) Int. Cl.: A61B 8/14, A61B 10/02

(54) **BIOPSY DEVICE WITH MANIFOLD ALIGNMENT FEATURE AND TISSUE SENSOR**
BIOPSIEVORRICHTUNG MIT EINER VERTEILERAUSRICHTUNGSFUNKTION UND GEWEBESENSOR
DISPOSITIF DE BIOPSIE AVEC ÉLÉMENT D'ALIGNEMENT DE COLLECTEUR ET DÉTECTEUR DE TISSU

(30) Priority: 03.05.2011 US 201113099497
(43) Date of publication of application: 12.03.2014
(73) Proprietor: Devicor Medical Products, Inc., Cincinnati, OH 45241 (US)
(72) Inventor: MOORE, Kyle, P., Mason OH 45040 (US); CRAIG, Harold, W., Cincinnati OH 45249 (US); SPEEG, Trevor, W.v., Williamsburg OH 45176 (US); TANGHAL, Emmanuel, V., Mason OH 45040 (US); MESCHER, Patrick, A., Bellbrook OH 45305 (US); MUMAW, Daniel, J., Milford OH 45150 (US); KOHNHORST, Lois, L., Gahanna OH 43230 (US); DODD, Kathryn, M., Cincinnati OH 45208 (US)
(74) Representative: Tunstall, Christopher Stephen
(86) International application number: PCT/US2012/034169
(87) International publication number: WO 2012/151053

(56) References cited:
- WO-A2-2007/112751
- US-A1- 2008 132 805
- US-A1- 2008 132 805
- US-A1- 2008 221 480
- US-A1- 2008 221 480
- US-A1- 2010 081 928
- US-A1- 2010 081 964
- US-A1- 2010 160 817
- US-A1- 2011 021 948

## Description

### BACKGROUND

Biopsy samples have been obtained in a variety of ways in various medical procedures using a variety of devices. Biopsy devices may be used under stereotactic guidance, ultrasound guidance, MRI guidance, PEM guidance, BSGI guidance, or otherwise. For instance, some biopsy devices may be fully operable by a user using a single hand, and with a single insertion, to capture one or more biopsy samples from a patient. In addition, some biopsy devices may be tethered to a vacuum module and/or control module, such as for communication of fluids (e.g., pressurized air, saline, atmospheric air, vacuum, etc.), for communication of power, and/or for communication of commands and the like. Other biopsy devices may be fully or at least partially operable without being tethered or otherwise connected with another device.

Merely exemplary biopsy devices are disclosed in U.S. Pat. No. 5,526,822, entitled "Method and Apparatus for Automated Biopsy and Collection of Soft Tissue," issued June 18, 1996; U.S. Pat. No. 6,086,544, entitled "Control Apparatus for an Automated Surgical Biopsy Device," issued July 11, 2000; U.S. Pub. No. 2003/0109803, entitled "MRI Compatible Surgical Biopsy Device," published June 12, 2003; U.S. Pub. No. 2006/0074345, entitled "Biopsy Apparatus and Method," published April 6, 2006; U.S. Pub. No. 2007/0118048, entitled "Remote Thumbwheel for a Surgical Biopsy Device," published May 24, 2007; U.S. Pub. No. 2008/0214955, entitled "Presentation of Biopsy Sample by Biopsy Device," published September 4, 2008; U.S. Pub. No. 2009/0171242, entitled "Clutch and Valving System for Tetherless Biopsy Device," published July 2, 2009; U.S. Pub. No. 2010/0152610, entitled "Hand Actuated Tetherless Biopsy Device with Pistol Grip," published June 17, 2010; U.S. Pub. No. 2010/0160819, entitled "Biopsy Device with Central Thumbwheel," published June 24, 2010; U.S. Pub. No. 2010/0317997, entitled "Tetherless Biopsy Device with Reusable Portion," published December 16, 2010; and U.S. Non-Provisional Patent App. No. 12/953,715, entitled "Handheld Biopsy Device with Needle Firing," filed November 24, 2010.

US 2008/0221480 A1 describes a biopsy device used to obtain and store multiple tissue samples.

While several systems and methods have been made and used for obtaining a biopsy sample, it is believed that no one prior to the inventors has made or used the invention described in the appended claims.

### BRIEF DESCRIPTION OF THE DRAWINGS

While the specification concludes with claims which particularly point out and distinctly claim the invention, it is believed the present invention will be better understood from the following description of certain examples taken in conjunction with the accompanying drawings, in which like reference numerals identify the same elements. In the drawings some components or portions of components are shown in phantom as depicted by broken lines.
FIG. 1 depicts a perspective view of an exemplary biopsy device;
FIG. 2 depicts a perspective view of a probe portion of the biopsy device of FIG. 1 separated from a holster portion of the biopsy device of FIG. 1;
FIG. 3 depicts a top perspective view of the probe portion of the biopsy device of FIG. 1, with a top housing piece removed;
FIG. 4 depicts an exploded perspective view of cutter actuation components of the probe of FIG. 3;
FIG. 5 depicts a side cross-sectional view of needle hub and manifold components of the probe of FIG. 3;
FIG. 6A depicts a side cross-sectional view of the tissue sample holder of the probe of FIG. 3, with the cutter at a distal position before a cutting cycle begins;
FIG. 6B depicts a top cross-sectional view of a valve assembly of the probe of FIG. 3, taken along line 6B-6B of FIG. 6A, with the cutter at a distal position before a cutting cycle begins;
FIG. 6C depicts a side cross-sectional view of cutter actuation components, as well as needle hub and manifold components, of the probe of FIG. 3, with the cutter at a distal position before a cutting cycle begins;
FIG. 6D depicts a side cross-sectional view of the cutter and needle of the probe of FIG. 3, with the cutter at a distal position before a cutting cycle begins;
FIG. 7A depicts a side cross-sectional view of the tissue sample holder of the probe of FIG. 3, with the cutter at a partially retracted position during a first stage of a cutting cycle;
FIG. 7B depicts a top cross-sectional view of a valve assembly of the probe of FIG. 3, taken along line 7B-7B of FIG. 7A, with the cutter at a partially retracted position during a first stage of a cutting cycle;
FIG. 7C depicts a side cross-sectional view of cutter actuation components, as well as needle hub and manifold components, of the probe of FIG. 3, with the cutter at a partially retracted position during a first stage of a cutting cycle;
FIG. 7D depicts a side cross-sectional view of the cutter and needle of the probe of FIG. 3, with the cutter at a partially retracted position during a first stage of a cutting cycle;
FIG. 8A depicts a side cross-sectional view of the tissue sample holder of the probe of FIG. 3, with the cutter at a retracted, proximal position;
FIG. 8B depicts a top cross-sectional view of a valve assembly of the probe of FIG. 3, taken along line 8B-8B of FIG. 8A, with the cutter at a retracted, proximal position;
FIG. 8C depicts a side cross-sectional view of cutter actuation components, as well as needle hub and manifold components, of the probe of FIG. 3, with the cutter at a retracted, proximal position;
FIG. 8D depicts a side cross-sectional view of the cutter and needle of the probe of FIG. 3, with the cutter at a retracted, proximal position;
FIG. 9A depicts a side cross-sectional view of the tissue sample holder of the probe of FIG. 3, with the cutter at a partially advanced position during a second stage of a cutting cycle;
FIG. 9B depicts a top cross-sectional view of a valve assembly of the probe of FIG. 3, taken along line 9B-9B of FIG. 9A, with the cutter at a partially advanced position during a second stage of a cutting cycle;
FIG. 9C depicts a side cross-sectional view of cutter actuation components, as well as needle hub and manifold components, of the probe of FIG. 3, with the cutter at a partially advanced position during a second stage of a cutting cycle;
FIG. 9D depicts a side cross-sectional view of the cutter and needle of the probe of FIG. 3, with the cutter at a partially advanced position during a second stage of a cutting cycle;
FIG. 10 depicts a perspective view of the tissue sample holder of the probe of FIG. 3, viewed from a proximal side;
FIG. 11 depicts an exploded perspective view of the tissue sample holder of FIG. 10, and a tissue sample holder rotation mechanism of the probe of FIG. 3;
FIG. 12 depicts a perspective view of the tissue sample holder of the probe of FIG. 3, viewed from a distal side;
FIG. 13 depicts a perspective view of the holster portion of the biopsy device of FIG. 1, with a top housing piece removed;

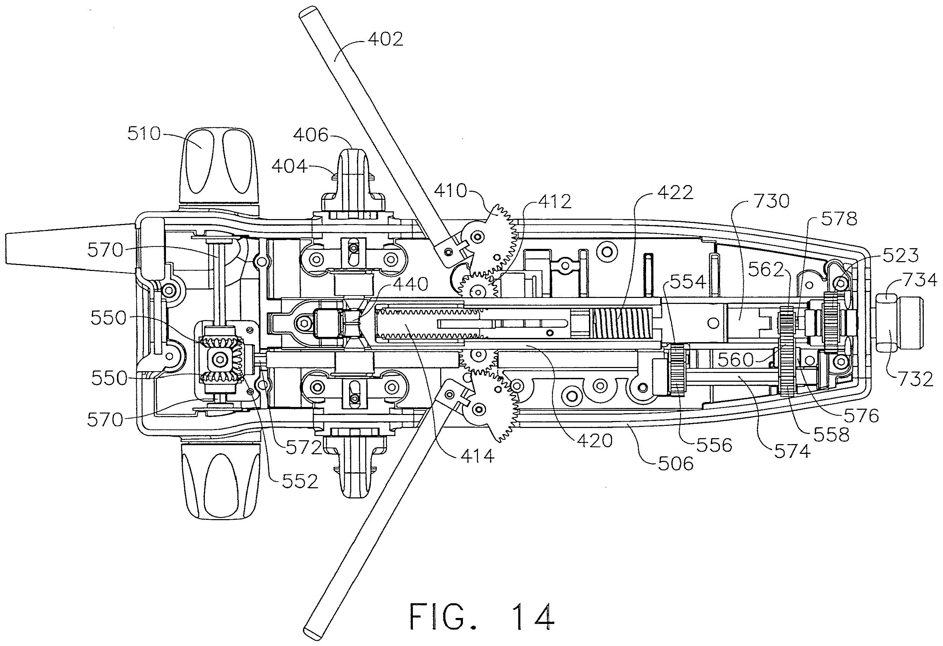

FIG. 14 depicts a top plan view of the holster of FIG. 13, with the top housing piece and other components removed;
FIG. 15A depicts a perspective view of needle firing mechanism components of the holster of FIG. 14, with the needle firing mechanism in a cocked and armed configuration;
FIG. 15B depicts a perspective view of the needle firing mechanism components of FIG. 15A, with the needle firing mechanism in a fired configuration;
FIG. 16 depicts a schematic diagram of an exemplary biopsy device having an integral tissue sample sensor and an integral indicator;
FIG. 17 depicts a schematic diagram of an exemplary biopsy device having an integral tissue sample sensor and a remote indicator; and
FIG. 18 depicts an exemplary user interface.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

### I. Overview of Exemplary Biopsy Device

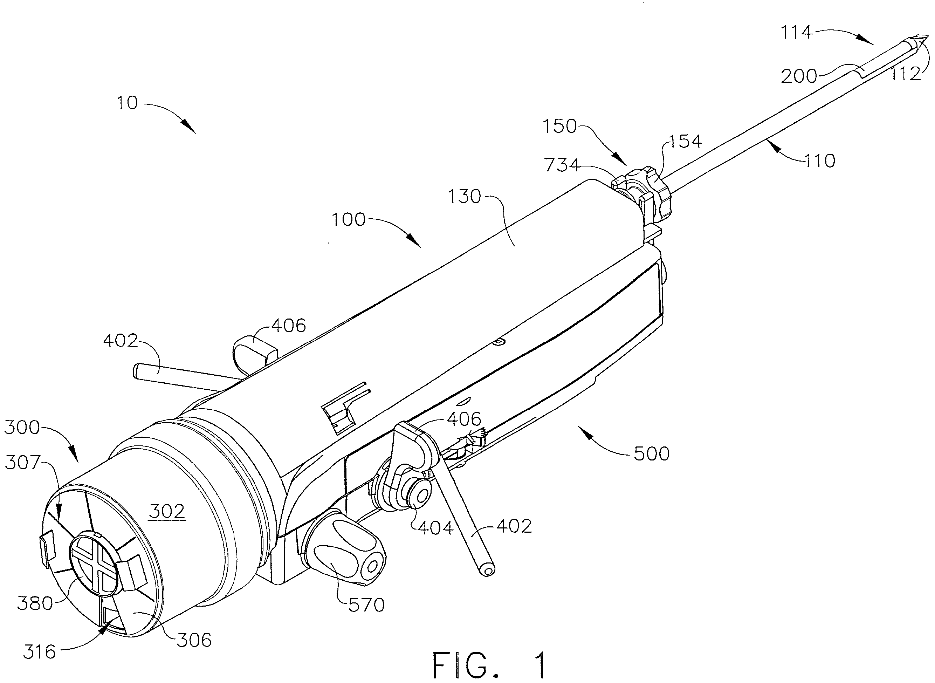

FIGS. 1-2 show an exemplary biopsy device (10). Biopsy device (10) of this example comprises a probe (100) and a holster (500). A needle (110) extends distally from probe (100), and is inserted into a patient's tissue to obtain tissue samples as will be described in greater detail below. These tissue samples are deposited in a tissue sample holder (300) at the proximal end of probe (100), as will also be described in greater detail below. It should also be understood that the use of the term "holster" herein should not be read as requiring any portion of probe (100) to be inserted into any portion of holster (500). While prongs (102) are used to removably secure probe (100) to holster (500) in the present example, it should be understood that a variety of other types of structures, components, features, etc. (e.g., bayonet mounts, latches, clamps, clips, snap fittings, etc.) may be used to provide removable coupling of probe (100) and holster (500). Furthermore, in some biopsy devices (10), probe (100) and holster (500) may be of unitary or integral construction, such that the two components cannot be separated. By way of example only, in versions where probe (100) and holster (500) are provided as separable components, probe (100) may be provided as a disposable component, while holster (500) may be provided as a reusable component. Still other suitable structural and functional relationships between probe (100) and holster (500) will be apparent to those of ordinary skill in the art in view of the teachings herein.

Some variations of biopsy device (10) may include one or more sensors (not shown), in probe (100) and/or in holster (500), that is/are configured to detect when probe (100) is coupled with holster (500). Such sensors or other features may further be configured to permit only certain types of probes (100) and holsters (500) to be coupled together. In addition or in the alternative, such sensors may be configured to disable one or more functions of probes (100) and/or holsters (500) until a suitable probe (100) and holster (500) are coupled together. Of course, such sensors and features may be varied or omitted as desired.

In some versions, biopsy device (10) includes a vacuum source (not shown), such as a conventional vacuum pump. By way of example only, a vacuum source may be incorporated into probe (100), incorporated into holster (500), and/or be a separate component altogether. In versions where a vacuum source is separate from probe (100) and holster (500), the vacuum source may be coupled with probe (100) and/or holster (500) via one or more conduits such as flexible tubing. In some versions, a vacuum source is in fluid communication with tissue sample holder (300) and needle (110). Thus, a vacuum source may be activated to draw tissue into lateral aperture (114) of needle (110). Tissue sample holder (300) is also in fluid communication with cutter (200). A vacuum source may thus also be activated to draw severed tissue samples through the hollow interior of cutter (200) and into tissue sample holder (300). By way of example only, a vacuum source may be provided in accordance with the teachings of U.S. Pub. No. 2008/0214955, the disclosure of which is incorporated by reference herein. In addition or in the alternative, a vacuum source may be provided in accordance with the teachings of U.S. Non-Provisional Patent App. No. 12/953,715. As yet another merely illustrative example, a vacuum source may be provided in accordance with the teachings of U.S. Non-Provisional Patent App. No. 12/709,695, entitled "Biopsy Device with Auxiliary Vacuum Source," filed February 22, 2010. Still other suitable ways in which a vacuum source may be provided will be apparent to those of ordinary skill in the art in view of the teachings herein. It should also be understood that a vacuum source may simply be omitted, if desired.

Biopsy device (10) of the present example is configured to mount to a table or fixture, and be used under stereotactic guidance. Of course, biopsy device (10) may instead be used under ultrasound guidance, MRI guidance, PEM guidance, BSGI guidance, or otherwise. It should also be understood that biopsy device (10) may be sized and configured such that biopsy device (10) may be operated by a single hand of a user. In particular, a user may grasp biopsy device (10), insert needle (100) into a
patient's breast, and collect one or a plurality of tissue samples from within the patient's breast, all with just using a single hand. Alternatively, a user may grasp biopsy device (10) with more than one hand and/or with any desired assistance. In some settings, the user may capture a plurality of tissue samples with just a single insertion of needle (110) into the patient's breast. Such tissue samples may be pneumatically deposited in tissue sample holder (300), and later retrieved from tissue sample holder (300) for analysis. While examples described herein often refer to the acquisition of biopsy samples from a patient's breast, it should be understood that biopsy device (10) may be used in a variety of other procedures for a variety of other purposes and in a variety of other parts of a patient's anatomy (e.g., prostate, thyroid, etc.). Various exemplary components, features, configurations, and operabilities of biopsy device (10) will be described in greater detail below; while other suitable components, features, configurations, and operabilities will be apparent to those of ordinary skill in the art in view of the teachings herein.

### II. Exemplary Probe

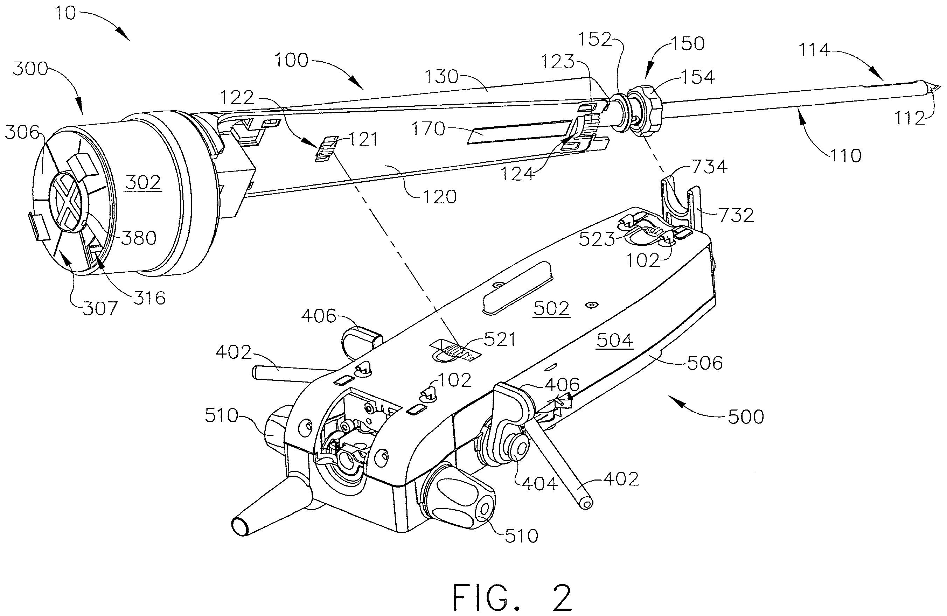

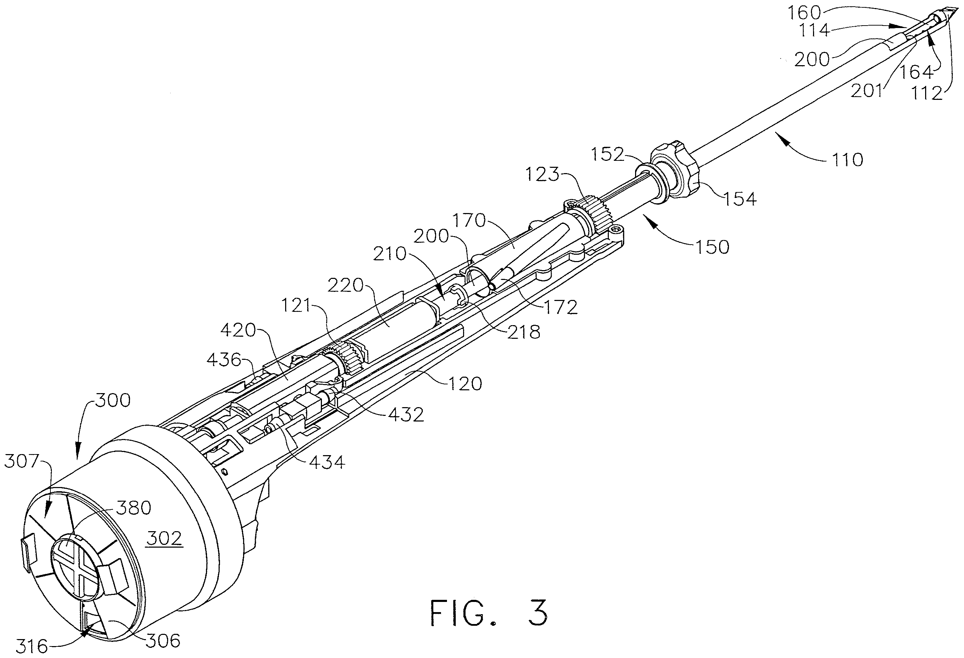

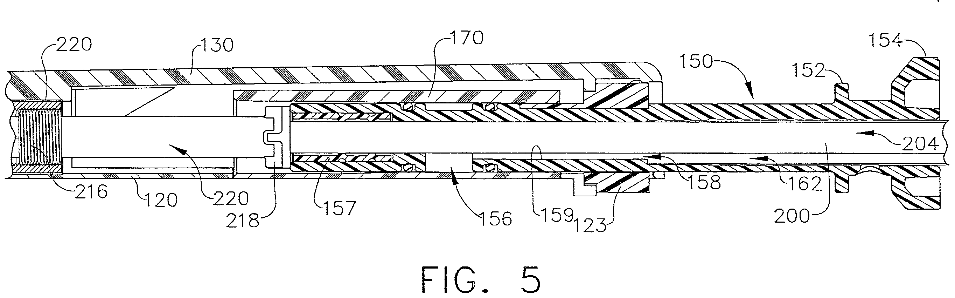

FIGS. 3-12 show probe (100) of the present example in greater detail. As noted above, probe (100) includes a distally extending needle (110). Probe (100) also includes a chassis (120) and a top housing (130), which are fixedly secured together. As best seen in FIG. 2, a gear (121) is exposed through an opening (122) in chassis (120), and is operable to drive a cutter actuation mechanism in probe (100) as will be described in greater detail below. As also seen in FIG. 2, another gear (123) is exposed through another opening (124) in chassis (120), and is operable to rotate needle (110) as will be described in greater detail below. Gear (121) of probe (100) meshes with exposed gear (521) of holster (500) when probe (100) and holster (500) are coupled together. Similarly, gear (123) of probe (100) meshes with exposed gear (523) of holster (500) when probe (100) and holster (500) are coupled together.

### A. Exemplary Needle

Needle (110) of the present example is shown in FIGS. 3, 5 AND 6D, 7D, 8D, 9D. As shown, needle (110) includes a piercing tip (112), a lateral aperture (114) located proximal to tip (112), and a hub member (150). Tissue piercing tip (112) is configured to pierce and penetrate tissue, without requiring a high amount of force, and without requiring an opening to be pre-formed in the tissue prior to insertion of tip (112). Alternatively, tip (112) may be blunt (e.g., rounded, flat, etc.) if desired. Tip (112) may also be configured to provide greater echogenicity than other portions of needle (110), providing enhanced visibility of tip (112) under ultrasound imaging. By way of example only, tip (112) may be configured in accordance with any of the teachings in U.S. Non-Provisional Pat, App. No. 12/875,200, entitled "Echogenic Needle for Biopsy Device," filed September 3, 2010. Other suitable configurations that may be used for tip (112) will be apparent to those of ordinary skill in the art in view of the teachings herein.

Lateral aperture (114) is sized to receive prolapsed tissue during operation of device (10). A hollow tubular cutter (200) having a sharp distal edge (201) is located within needle (110). Cutter (200) is operable to rotate and translate relative to needle (110) and past lateral aperture (114) to sever a tissue sample from tissue protruding through lateral aperture (114). For instance, cutter (200) may be moved from an extended position to a retracted position, thereby "opening" lateral aperture (114) to allow tissue to protrude therethrough; then from the retracted position back to the extended position to sever the protruding tissue. Examples of such operation are described in greater detail below. While lateral aperture (114) is shown oriented in an upward position in FIG. 1, it should be understood that needle (110) may be rotated to orient lateral aperture (114) at any desired angular position about the longitudinal axis of needle (110). Such rotation of needle (110) is facilitated in the present example by hub member (150), which will be described in greater detail below.

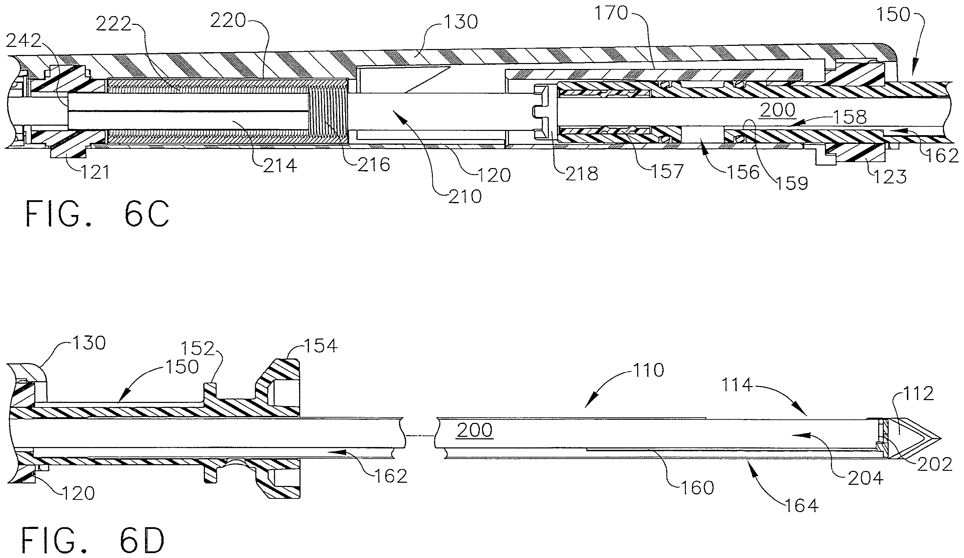

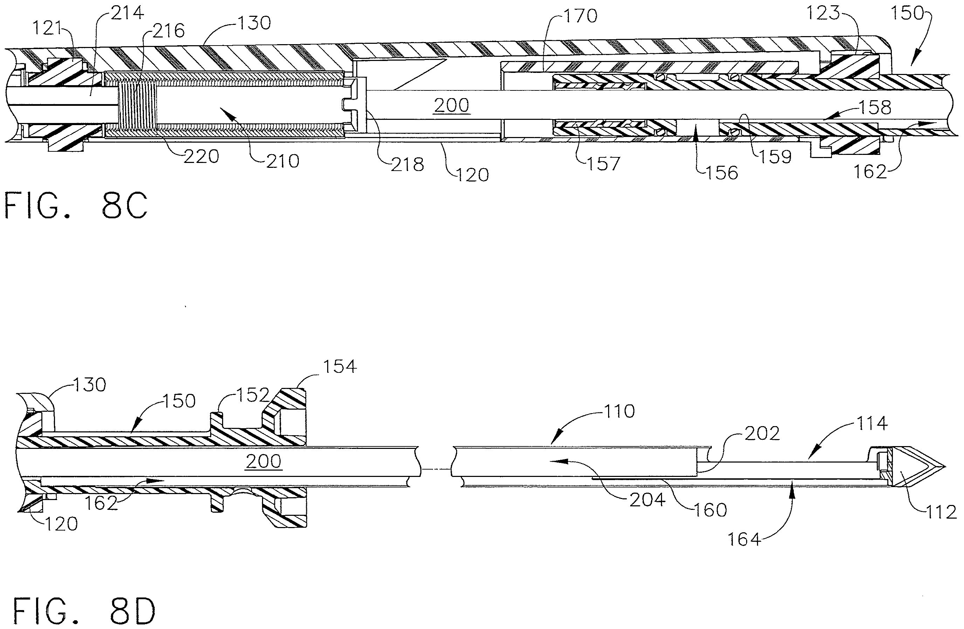

As best seen in FIGS. 6D, 7D, 8D, and 9D, needle (110) also includes a longitudinal wall (160) extending proximally from the proximal portion of tip (112). While wall (160) does not extend along the full length of needle (110) in this example, it should be understood that wall (160) may extend the full length of needle (110) if desired. Wall (160) of the present example proximally terminates at a longitudinal position that is just proximal to the longitudinal position of distal cutting edge (202) of cutter (200) when cutter (200) is in a proximal position (see FIG. 8D). Thus, wall (160)
and cutter (200) together define a second lumen (162) that is lateral to and parallel to cutter (200). Of course, wall (160) may alternatively proximally terminate at a longitudinal position that is just distal to the longitudinal position of distal cutting edge (202) of cutter (200) when cutter (200) is in a proximal position; or wall (160) may terminate at any other suitable longitudinal position. Wall (160) includes a plurality of openings (164) that provide fluid communication between second lumen (162) and the upper portion of needle (110), as well as fluid communication between second lumen (162) and the lumen (204) of cutter (200). For instance, as will be described in greater detail below, second lumen (162) may selectively provide atmospheric air to vent cutter lumen (204) during operation of biopsy device (10) as will be described in greater detail below. Openings (164) are arranged such that at least one opening (164) is located at a longitudinal position that is distal to the distal edge of lateral aperture (114). Thus, cutter lumen (204) and second lumen (162) may remain in fluid communication even when cutter (200) is advanced to a position where cutting edge (202) is located at a longitudinal position that is distal to the longitudinal position of the distal edge of lateral aperture (114) (se FIG. 6D). Of course, as with any other component described herein, any other suitable configurations may be used.

Hub member (150) of the present example is overmolded about needle (110), such that hub member (150) and needle (110) rotate and translate unitarily with each other. By way of example only, needle (110) may be formed of metal, and hub member (150) may be formed of a plastic material that is overmolded about needle (110) to unitarily secure and form hub member (150) to needle (110). Hub member (150) and needle (110) may alternatively be formed of any other suitable material(s), and may be secured together in any other suitable fashion. Hub member (150) includes an annular flange (152) and a thumbwheel (154). Gear (123) is slidably and coaxially disposed on a proximal portion (150) of hub member (150) and is keyed to hub member (150), such that rotation of gear (123) will rotate hub member (150) and needle (110); yet hub member (150) and needle (110) may translate relative to gear (123). Gear (123) is rotatably driven by gear (523), as will be described in greater detail below. Alternatively, needle (110) may be rotated by rotating thumbwheel (154). Various other suitable ways in which manual rotation of needle (110) may be provided will be apparent to those of ordinary skill in the art in view of the teachings herein. It should also be understood that rotation of needle (110) may be automated in various ways, including but not limited to the various forms of automatic needle rotation described in various references that are cited herein. Examples of how needle (110) may be translated longitudinally relative to chassis (120) and top housing (130), particularly by a needle firing mechanism, will be described in greater detail below.

It should be understood that, as with other components described herein, needle (110) may be varied, modified, substituted, or supplemented in a variety of ways; and that needle (110) may have a variety of alternative features, components, configurations, and functionalities. A plurality of external openings (not shown) may also be formed in needle (110), and may be in fluid communication with a lumen of needle (110) that is lateral to cutter (500). For instance, such external openings may be configured in accordance with the teachings of U.S. Pub. No. 2007/0032742, entitled "Biopsy Device with Vacuum Assisted Bleeding Control," published February 8, 2007. Cutter (200) may also include one or more side openings (not shown). Of course, as with other components described herein, such external openings in needle (110) and cutter (200) are merely optional. As yet another merely illustrative example, needle (110) may be constructed in accordance with the teachings of U.S. Pub. No. 2008/0214955, and/or in accordance with the teachings of any other reference cited herein.

It should be understood that, as with other components described herein, needle (110) may be varied, modified, substituted, or supplemented in a variety of ways; and that needle (110) may have a variety of alternative features, components, configurations, and functionalities. A plurality of external openings (not shown) may also be formed in needle (110), and may be in fluid communication with second lumen (162). For instance, such external openings may be configured in accordance with the teachings of U.S. Pub. No. 2007/0032742, entitled "Biopsy Device with Vacuum Assisted Bleeding Control," published February 8, 2007.
Cutter (200) may also include one or more side openings (not shown). Of course, as with other components described herein, such external openings in needle (110) and cutter (200) are merely optional. As another merely illustrative example, needle (110) may simply lack second lumen (162) altogether in some versions. Other suitable alternative versions, features, components, configurations, and functionalities of needle (110) will be apparent to those of ordinary skill in the art in view of the teachings herein.

### B. Exemplary Cutter Actuation Mechanism

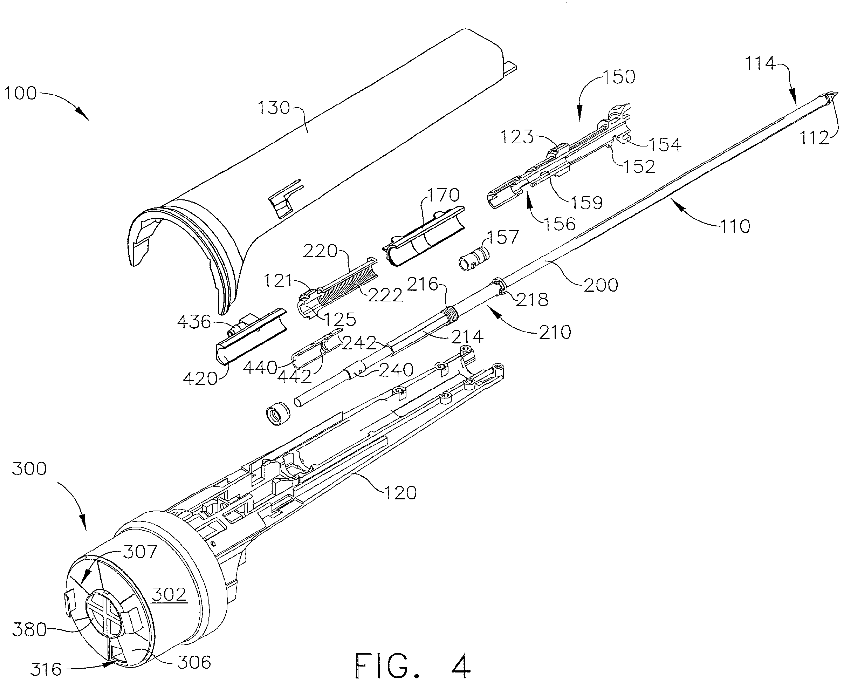

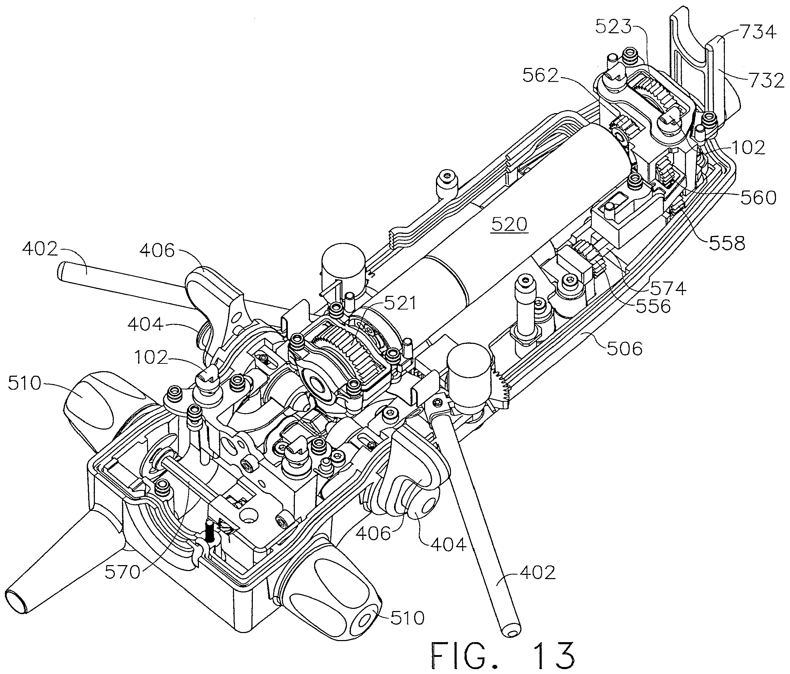

As noted above, cutter (200) is operable to rotate and translate relative to needle (110) and past lateral aperture (114) to sever a tissue sample from tissue protruding through lateral aperture (114). This action of cutter (200) is provided by a cutter actuation mechanism. The cutter actuation mechanism is also operable to retract cutter (200) proximally to ready cutter (200) for firing. Components of the cutter actuation mechanism of the present example are shown in FIGS. 4, 6C, 7C, 8C, 9C, and 13. These components are positioned mainly in probe (100) in the present example, though it should be understood that the components may be positioned mainly in holster (500) and/or both in probe (100) and holster (500). The cutter actuation mechanism includes meshing gears (121, 521), with gear (521) being directly driven by motor (520). Motor (520) is located in holster (500) in the present example, though it should be understood that motor (520) may alternatively be located in probe (100) and/or elsewhere.

Gear (121) is slidably disposed about a cutter sleeve or overmold (210). Both of these components (121, 210) are coaxially aligned with cutter (200). Furthermore, cutter overmold (210) is fixedly secured to cutter (200), such that cutter overmold (210) and cutter (200) will rotate and translate unitarily together in the present example. By way of example only, cutter (200) may be formed of metal, and cutter overmold (210) may be formed of a plastic material that is overmolded about cutter (200) to unitarily secure and form cutter overmold (210) to cutter (200). Cutter overmold (210) and cutter (200) may alternatively be formed of any other suitable material(s), and may be secured together in any other suitable fashion. Cutter overmold (210) includes a proximal portion having external flats (214), a threaded intermediate section (216), and a distal stop member (218).

A nut (220) is positioned coaxially about cutter overmold (210) and cutter (200). Nut (220) is also fixedly secured relative to chassis (120) and top housing (130), such that nut (220) neither rotates nor translates relative to chassis (120) or top housing (130). Nut (220) includes internal threading (222) that complements the threading of threaded intermediate section (216) of cutter overmold. This threading has a fine pitch in the present example. It should be understood that, due to interaction between the complementary threading, rotation of cutter overmold (210) relative to nut (220) results in translation of cutter ovemold (210), and therefore translation of cutter (200). Gear (121) has internal flats (125) that complement external flats (214) of cutter overmold (210), such that cutter overmold (210) and cutter (200) rotate when gear (121) is rotated. Furthermore, flats (125, 214) permit cutter overmold (210) to translate relative to gear (121). While flats (125, 214) define octagonal profiles in the present example, it should be understood that other suitable structures may be used, including but not limited to hexagonal flats, complementary keys and keyways, etc. It should also be understood that the longitudinal position of gear (121) remains substantially constant relative chassis (120) and top housing (130) during operation of biopsy device (10) of the present example.

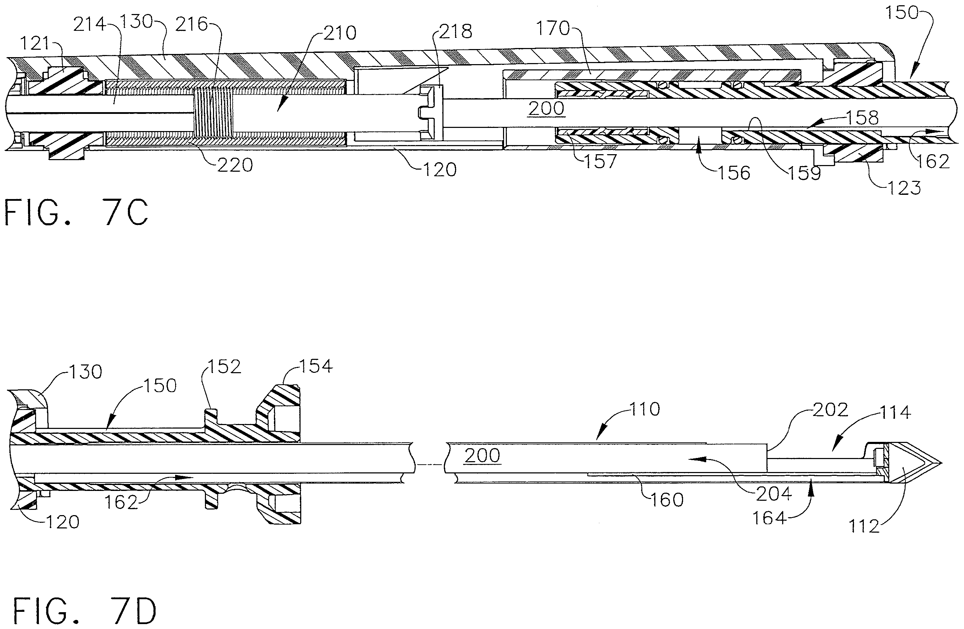

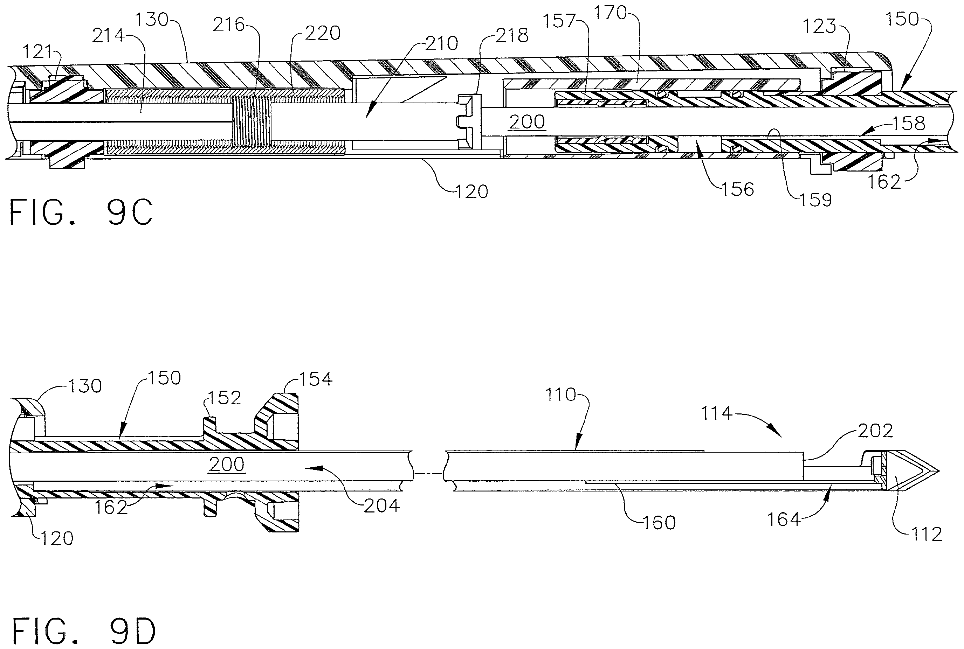

Motor (520) may be activated to rotate gear (521) in one direction to retract cutter (200) proximally; then in the opposite direction to advance cutter (200) distally. For instance, FIG. 6D shows cutter (200) starting out at a distal position (with lateral aperture (114) being effectively closed); FIG. 7D shows cutter (200) at a partially retracted position (with lateral aperture (114) being effectively partially opened); and FIG. 8D shows cutter (200) at a retracted position (with lateral aperture (114) being effectively opened). Thus motor (520) rotates drive gear (521) in one direction to transition from the distal position of FIG. 6D to the retracted position of FIG. 8D. With motor (520) being thereafter reversed, FIG. 9D shows cutter (200) at a partially advanced position, advancing distally back toward the distal position shown in FIG. 6D. It should be understood that when cutter (200) is retracted to the proximal position shown in FIG. 8D, tissue may prolapse through lateral aperture (114) under the force of gravity, due to internal pressure of the tissue (e.g., caused by displacement of the tissue upon insertion of needle (110), etc.), caused by manual external palpation of the patient's breast by the physician, and/or under the influence of vacuum provided through cutter lumen (204) as described elsewhere herein. When cutter (200) is then advanced distally, distal edge (202) severs tissue protruding through lateral aperture (114). This severed tissue is captured within cutter lumen (204). A vacuum applied through cutter lumen (204) (as described herein or otherwise) will be encountered by the proximal face of a severed tissue sample within cutter lumen (204). A vent may be applied through second lumen (162) of needle (110), which may be communicated to the distal face of the severed tissue sample via openings (164), providing a pressure differential for the severed tissue sample. This pressure differential may facilitate proximal transport of the severed tissue sample through cutter lumen (204), whereby the severed tissue sample eventually reaches tissue sample holder (300) as described elsewhere herein. Alternatively, tissue samples severed by cutter (200) may be communicated proximally to tissue sample holder (300) or be otherwise dealt with in any other suitable fashion.

By way of example only, the cutter actuation mechanism of biopsy device (10) may be constructed in accordance with the teachings of U.S. Pub. No. 2008/0214955. As another merely illustrative example, the cutter actuation mechanism may be constructed in accordance with the teachings of U.S. Pub. No. 2010/0317997. As yet another merely illustrative example, the cutter actuation mechanism may be constructed in accordance with the teachings of U.S. Pub. No. 2010/0292607, entitled "Tetherless Biopsy Device with Self-Reversing Cutter Drive Mechanism," published November 18, 2010. Alternatively, cutter the actuation mechanism may be constructed in accordance with the teachings of any other reference cited herein. It should also be understood that biopsy device (10) may be configured such that cutter (200) does not translate (e.g., such that cutter (200) merely rotates, etc.); or such that cutter (200) does not rotate (e.g., such that cutter (200) merely translates, etc.). As another merely
illustrative example, cutter (200) may be actuated pneumatically in addition to or in lieu of being actuated by mechanical components. Other suitable alternative versions, features, components, configurations, and functionalities of a cutter actuation mechanism will be apparent to those of ordinary skill in the art in view of the teachings herein.

### C. Exemplary Needle Valving Mechanism

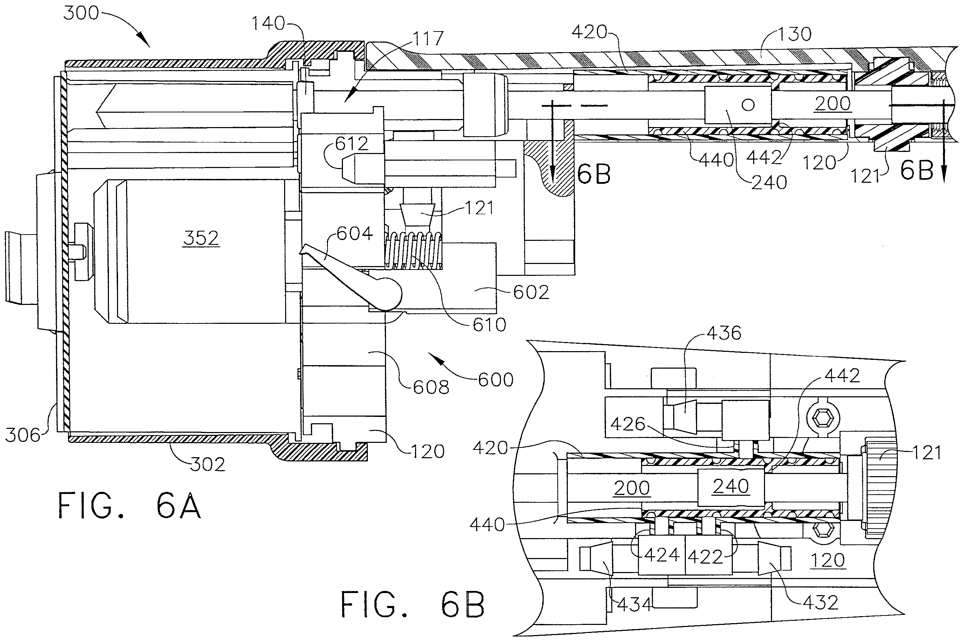

As shown in FIGS. 6B, 7B, 8B, and 9B, probe (100) further includes components that are operable to selectively vent or seal second lumen (162) of needle (110) relative to atmosphere. These components include a needle manifold (170), a vent sleeve (420), and a shuttle valve slider (440). As shown in FIGS. 3-5, needle manifold (170) is disposed around a proximal portion of hub member (150). Hub member (150) includes a transverse opening (156) that is positioned within the hollow interior of needle manifold (170). This transverse opening (156) is in fluid communication with second lumen (162) of needle (110) via a gap (158) defined between the exterior of cutter (200) and the inner diameter of an associated bore (159) of hub member (150). Thus, second lumen (162) is in fluid communication with the interior of manifold (170) via gap (158) and opening (156). A snap-in seal (157) is provided in a proximal portion of hub member (150), providing a dynamic seal around the exterior of cutter (200). As best seen in FIG. 3, a port (172) extends from manifold (170). Port (172) is in fluid communication with the hollow interior defined by manifold (170), such that port (172) is further in fluid communication with second lumen (162). In the present example, manifold (170) and hub member (150) are configured such that port (172) remains in fluid communication with second lumen (162) regardless of the translational position of needle (110) relative to chassis (120) and regardless of the rotational position of needle (110) relative to chassis (120). Other than opening (156), hub member (150) is sealed relative to manifold (170). Other than port (172) and opening (156), manifold (170) is sealed relative to other components of biopsy device (10) and relative to atmosphere.

Vent sleeve (420) is secured relative to chassis (120) and top housing (130), such that vent sleeve (420) does not move during operation of biopsy device (10); while shuttle valve slider (440) translates based on operational movement of cutter (200). Vent sleeve (420) includes a first transverse port (422), a second transverse port (424), and a third transverse port (426). A first coupling (432) is secured to first transverse port (422) and is in fluid communication therewith. A second coupling (434) is secured to second transverse port (424) and is in fluid communication therewith. A third coupling (436) is secured to third transverse port (436) and is in fluid communication therewith. First coupling (432) is coupled with port (172) of needle manifold (170) via a conduit (not shown) such as a flexible tube, etc. Second coupling (434) is in fluid communication with atmospheric air. In some versions a filter is provided on second coupling (434). Third coupling (436) is in fluid communication with a vacuum source (not shown), such as via flexible tubing, etc. Alternatively, either coupling (434, 436) may be in fluid communication with a source of saline, a source of pressurized fluid, and/or something else, etc.

Shuttle valve slider (440) of the present example is movable to vary the pneumatic state of first coupling (432) between the following three states - sealed, vacuum, or vented to atmosphere. Shuttle valve slider (440) is disposed coaxially about cutter (200), and has an inner diameter sized to permit shuttle valve slider (440) to longitudinally slide freely relative to cutter (200). Shuttle valve slider (440) also translates relative to vent sleeve (420), based on interaction between shuttle valve slider (440) and cutter (200). In particular, shuttle valve slider (440) includes an inner flange (442) that is pushed by components fixedly secured to cutter (200). One such component is a pusher (240), which is fixedly secured to a proximal portion of cutter (200). Another such component is the proximal end (242) of cutter overmold (210), which is fixedly secured to cutter (200) as described above. Pusher (240) pushes shuttle valve slider (440) distally by impinging against flange (442) when cutter (200) is advanced distally; while proximal end (242) pushes shuttle valve slider (440) proximally by impinging against flange (442) when cutter (200) is retracted proximally.

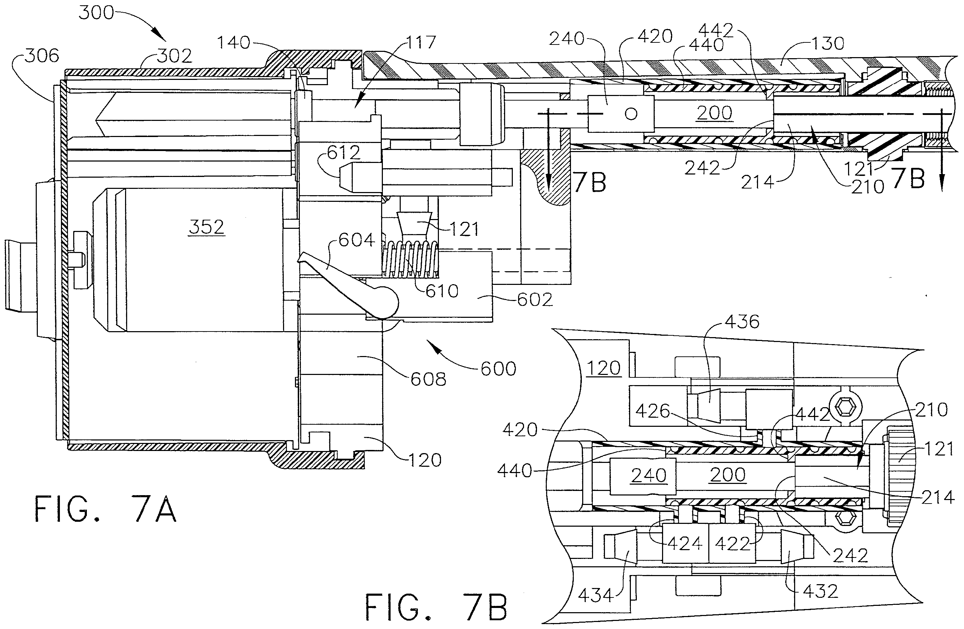

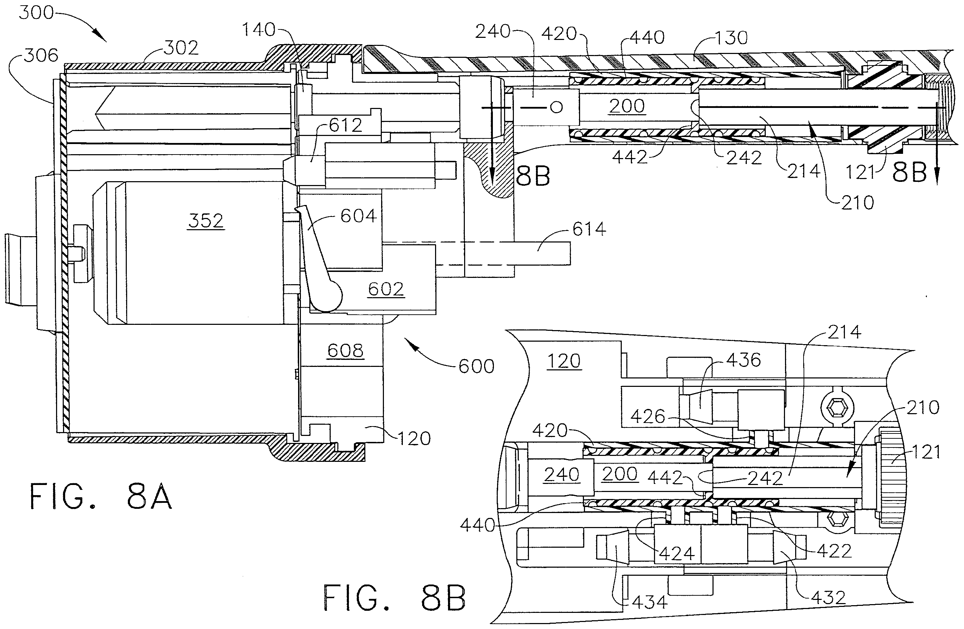

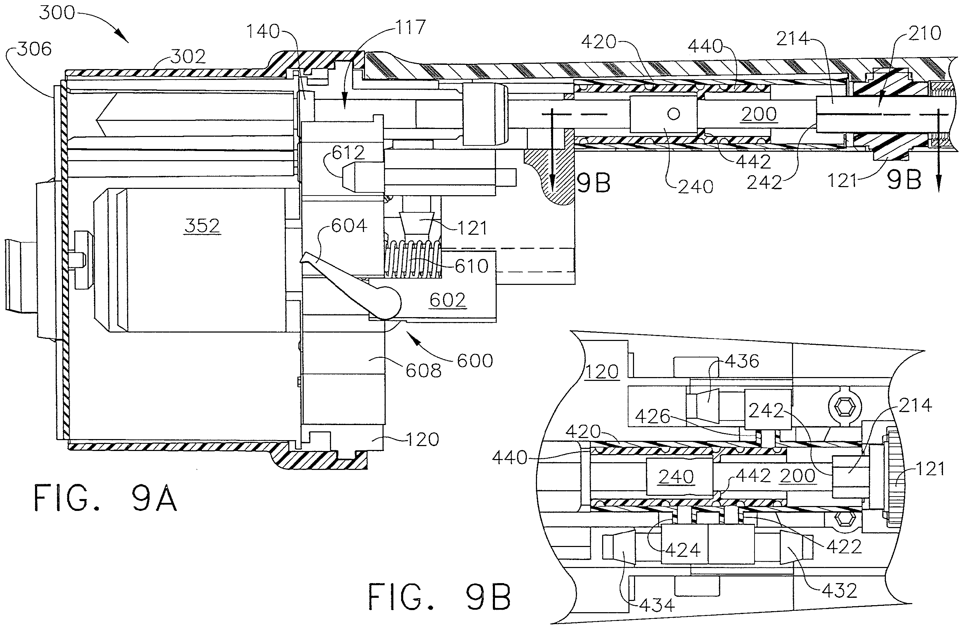

In the present example, the spacing between the pusher (240) and proximal end (242) is such that there is a certain degree of "lost motion" between cutter (200) and shuttle valve slider (440). In other words, there is a certain range of longitudinal travel of cutter (200) in either direction when neither pusher (240) nor proximal end (242) impinges against flange (442). This can be seen in the transition from the position shown in FIGS, 6A-6B to the position shown in FIGS. 7A-7B. During this proximal motion of cutter (200), shuttle valve slider (440) does not move. However, during the further proximal motion of cutter (200) shown in the transition from FIGS. 7A-7B to FIGS. 8A-8B, proximal end (242) pushes shuttle valve slider (440) proximally. Similarly, there is lost motion when cutter (200) is advanced distally from the position shown in FIGS. 8A-8B to the position shown in FIGS. 9A-9B, such that shuttle valve slider (440) does not move during this transition. As cutter (200) continues to move distally from the position shown in FIGS. 9A-9B back to the position shown in FIGS. 6A-6B, pusher (240) pushes shuttle valve slider (440) distally.

A plurality of o-rings (not shown) are disposed about the exterior of shuttle valve slider (440). These o-rings are spaced and positioned to selectively transition the pneumatic state of first coupling (432) between sealed, vacuum, or vented to atmosphere, based on the longitudinal position of shuttle valve slider (440) within vent sleeve (420). In particular, when cutter (200) is in the distal position shown in FIG. 6, shuttle valve slider (440) places first coupling (432) in fluid communication with second coupling (434), thereby venting second lumen (162) to atmosphere. As cutter (200) initially moves proximally to the partially retracted position shown in FIG. 7, shuttle valve slider (440) keeps first coupling (432) in fluid communication with second coupling (434), thereby continuing to vent second lumen (162) to atmosphere. As cutter (200) reaches the retracted position shown in FIG. 8, shuttle valve slider (440) is moved proximally, placing first coupling (432) in fluid communication with third coupling (436), thereby providing vacuum to second lumen (162). This may provide assistance in pulling tissue into aperture (114). It should be understood that shuttle valve slider (440) substantially seals first coupling (432) relative to both second coupling (434) and third coupling (436) during part of the proximal movement of shuttle valve slider (440) from the position shown in FIG. 7 to the position shown in FIG. 8. As cutter (200) is thereafter advanced distally to the partially advanced position shown in FIG. 9, shuttle valve slider (440) keeps first coupling (432) in fluid communication with third coupling (434), thereby continuing to communicate vacuum to second lumen (162). This may provide assistance in holding tissue in aperture (114) as the cutting edge (202) of cutter (200) severs the tissue. As cutter (200) ultimately reaches the distally advanced position shown in FIG. 6, shuttle valve slider (440) is eventually moved distally, placing first coupling (432) back in fluid communication with first coupling (434), thereby venting second lumen (162) to atmosphere again. Shuttle valve slider (440) substantially seals first coupling (432) relative to both second coupling (434) and third coupling (436) during part of the distal movement of shuttle valve slider (440) from the position shown in FIG. 9 to the position shown in FIG. 6.

In the present example, a vacuum is continuously communicated to cutter lumen (204) during all of the operational stages shown in FIGS. 6-9. This vacuum thus assists in drawing tissue into aperture (114). Furthermore, after cutter (200) has been advanced from the proximal position shown in FIG. 8 back to the distal position shown in FIG. 9 to sever a tissue sample from tissue protruding through aperture (114), a vacuum in cutter lumen (204) provides assistance in communicating the severed tissue sample proximally through cutter lumen (204) to tissue sample holder (300). In particular, a vacuum communicated form tissue sample holder (300) through cutter lumen (204) acts on the proximal face of a tissue sample within cutter lumen (204); while atmospheric air from second lumen (162) is communicated to the distal face of the tissue sample, thereby providing a pressure differential urging the tissue sample proximally through cutter lumen (204).

It should be understood that, as with other components described herein, the valving components described above may be varied, modified, substituted, or supplemented in a variety of ways; and that a valve mechanism may have a variety of alternative features, components, configurations, and functionalities. For instance, the valving components described above may be constructed and/or operable in accordance with the teachings of U.S. Pub. No. 2010/0317997, in accordance with the teachings of U.S. Non-Provisional Patent App. No. 12/953,715, or otherwise. In addition or in the alternative, valving may be provided by vacuum source (800) and/or a vacuum canister, such as is taught in U.S. Pub. No. 2008/0214955. Other suitable alternative versions, features, components, configurations, and functionalities of a valving system will be apparent to those of ordinary skill in the art in view of the teachings herein. It should also be understood that such valving may simply be omitted altogether, if desired.

### D. Exemplary Tissue Sample Holder

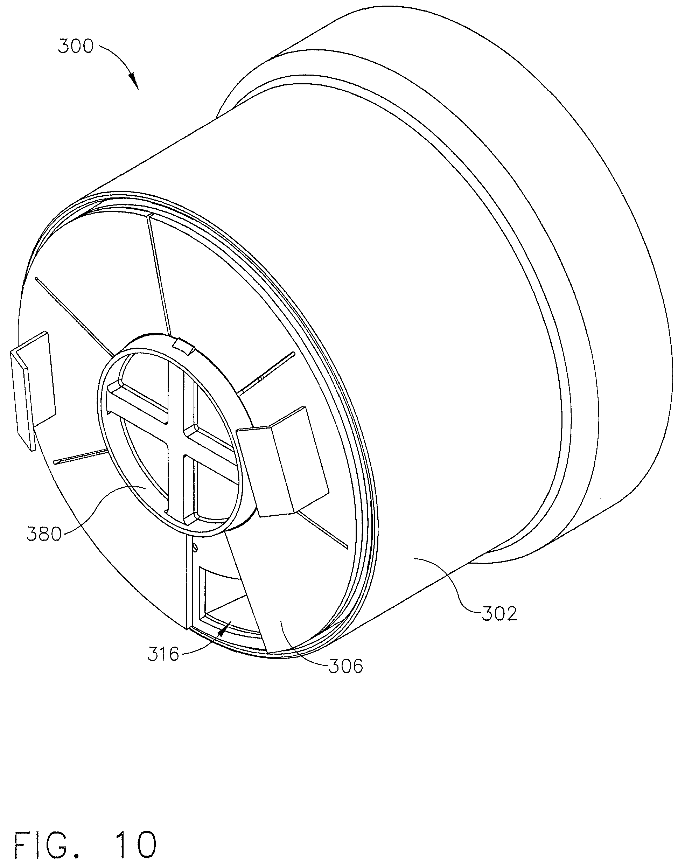

As shown in FIGS. 10-12, tissue sample holder (300) of the present example includes an outer shroud (302) and an interior housing (304). Housing (304) is configured to receive a removable tray (306), which defines a plurality of tissue sample chambers (345). As will be described in greater detail below, each tissue sample chamber (345) is configured to receive at least one tissue sample captured by cutter (200) and communicated proximally through cutter lumen (204). A pawl assembly (600) is provided for rotation of housing (304), to successively index tissue sample chambers (345) to cutter lumen (204), as will be described in greater detail below.

In the present example, outer shroud (302) has a cylindraceous shape, though any other suitable shapes or configurations may be used. Outer shroud (302) is configured to engage chassis (120) in a bayonet fashion, such that outer shroud (302) may be selectively removed from or secured to chassis (120). Other suitable configurations for providing selective engagement between outer shroud (302) and probe (100) will be apparent to those skilled in the art in view of the teachings herein. Shroud (302) is configured to cover interior housing (304), such that rotating or indexing interior housing (304) relative to chassis (120) will not rub against any external object. In particular, shroud (302) remains stationary relative to chassis (120) while housing (304) rotates within shroud (302). Shroud (302) of the present example is formed of a transparent material, enabling the user to visually inspect tissue samples in tissue sample holder (300) while tissue sample holder (300) is still coupled with chassis (120). For instance, a user may inspect tissue samples for color, size, and density (e.g., to the extent that a chamber (316, 345) is full of saline, etc.). Alternatively, shroud (302) may be translucent; opaque; a combination of translucent, opaque, and/or transparent; or have any other desired
properties. For instance, a translucent shroud (302) may prevent a patient from seeing blood in a tissue sample chamber (345). In the present example, shroud (302) is configured to permit tray (306) to be removed from housing (304) without having to first remove shroud (302). Still other ways in which shroud (302) may be configured and used will be apparent to those of ordinary skill in the art in view of the teachings herein. It should also be understood that, like other components described herein, shroud (302) is merely optional and may be omitted or varied in a number of ways if desired.

Housing (304) of the present example has a cylindraceous shape and comprises a plurality of radially extending interior walls (312). Radially extending walls (312) define a plurality of chambers (316). Each chamber (316) has a proximal end and a distal end. As shown in this example, housing (304) has thirteen chambers (316). However, housing (304) may have any other suitable number of chambers (316). The proximal end of each chamber (316) is configured to receive a portion of tray (306). The distal end of each chamber (316) is generally enclosed aside from an upper aperture (322) and a lower aperture (324) formed therethrough. When holder (300) and probe (100) are engaged, upper aperture (322) and lower aperture (324) of the chamber (316) that is located in the "12 o'clock position" are configured to respectively self-align with an upper o-ring (140) and a lower o-ring (142). O-rings (140, 142) are configured to provide a seal between chassis (120) and apertures (322, 324). In particular, chassis (120) has a first lumen (117) that is coaxially aligned with cutter lumen (204) and in fluid communication with cutter lumen (204). O-ring (140) provides a sealing fit between aperture (322) and first lumen (117). Accordingly, aperture (322) of a chamber (316) that is located in the "12 o'clock position" will be in fluid communication with cutter lumen (204) in this example. Tissue samples that are severed by cutter (200) may thus be communicated proximally through cutter lumen (204) (due to a pressure gradient as described above), through first lumen (117), through aperture (322), and into the chamber (316) that is located in the "12 o'clock position" in this example. Chassis (120) also has a second lumen (119) extending to a coupling (121), which is in fluid communication with a vacuum source (not shown). O-ring (142) provides a sealing fit between aperture (324) and that particular lumen. Accordingly, aperture (324) of a chamber (316) that is located in the "12 o'clock position" will be in fluid communication with the vacuum source. It should be understood that such a vacuum may be further communicated through aperture (322), and hence through cutter lumen (204), through apertures (344) formed in a tray (306) that is inserted in the chamber (316). It should therefore be understood that housing (304) may act as a manifold, such as by redirecting fluid communication, etc.

Chambers (316) also include guide rails (326) on the surface of walls (312). A set of guide rails (326) in each chamber (316) engage or support tray (306) upon tray (306) being received into chambers (316). In the example of tray (306) shown in FIGS. 10-11, a single tray (306) is configured to engage twelve chambers (316) of housing (304). The thirteenth chamber (316) is left open for insertion of a medical instrument, such as is disclosed in U.S. Pub. No. 2008/0214955 or otherwise. Of course, tray (306) may alternatively occupy any other number of chambers (316). Each chamber (345) of tray (306) has an associated tissue sample entry opening (347) that is aligned with the respective upper aperture (322) and that selectively aligns with lumens (117, 204). Tray (306) is formed of a flexible material and includes a plurality of joints (307), such that portions of tray (306) may bend or flex at such joints (307), allowing tray (306) to bend to conform to the round shape of housing (304) and also be flattened out after removal from chambers (316). A gasket (370) is provided between tray (306) and housing (304). A removable cap member (380) is removably secured to housing (304), and is sized, shaped, and positioned to help retain tray (306) against housing (304). Other suitable variations of tray (306) and housing (304) will be apparent to those of ordinary skill in the art. For example, tray (306) may be configured to only engage a single corresponding chamber (316) within housing (304), such that a plurality of trays (306) may be inserted in housing (304).

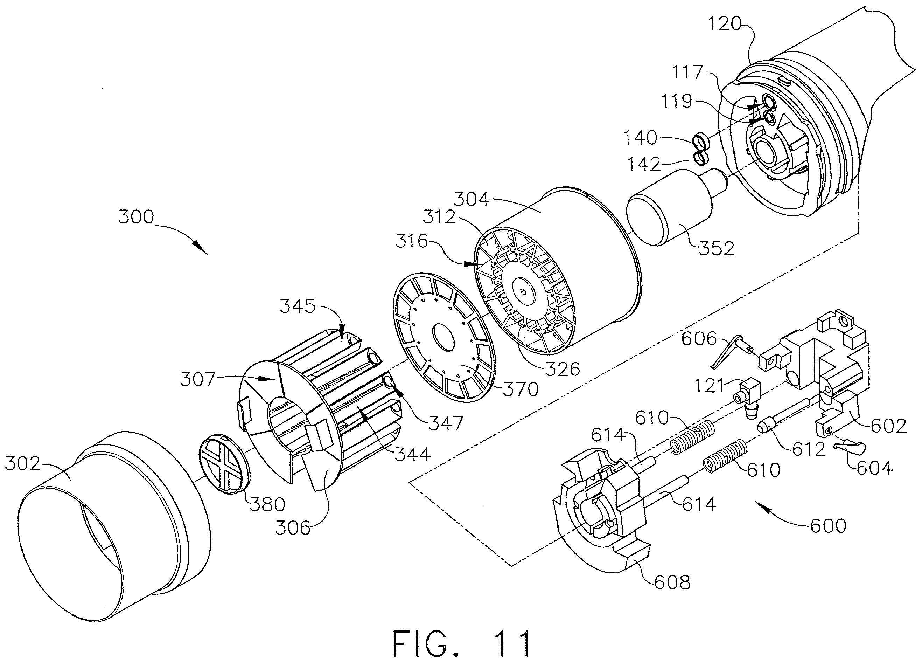

Housing (304) is secured to shaft (352), which is inserted in bore (305) of housing (304), and which freely rotates relative to chassis (120). Housing (304) thus rotates relative to chassis (120), about an axis defined by shaft (352). This rotation is provided by pawl assembly (600) in the present example. Components of pawl assembly (600) are best seen in FIG. 11, while operation of pawl assembly (600) is best seen in FIGS. 6A, 7A, 8A, 9A. Pawl assembly (600) of this example includes a reciprocating block (602), a pair of pawls (604, 606), a base member (608), a pair of coil springs (610), and an alignment pin (612). Base member (608) is fixedly secured to chassis (120). Block (602) is mounted on posts (614) that extend distally from block (602). Springs (610) are coaxially positioned about posts (614), and resiliently bias block (602) to a distal position relative to base member (608). Pawls (604, 606) are pivotally coupled with base member (608). Alignment pin (612) is fixedly secured to block (602).

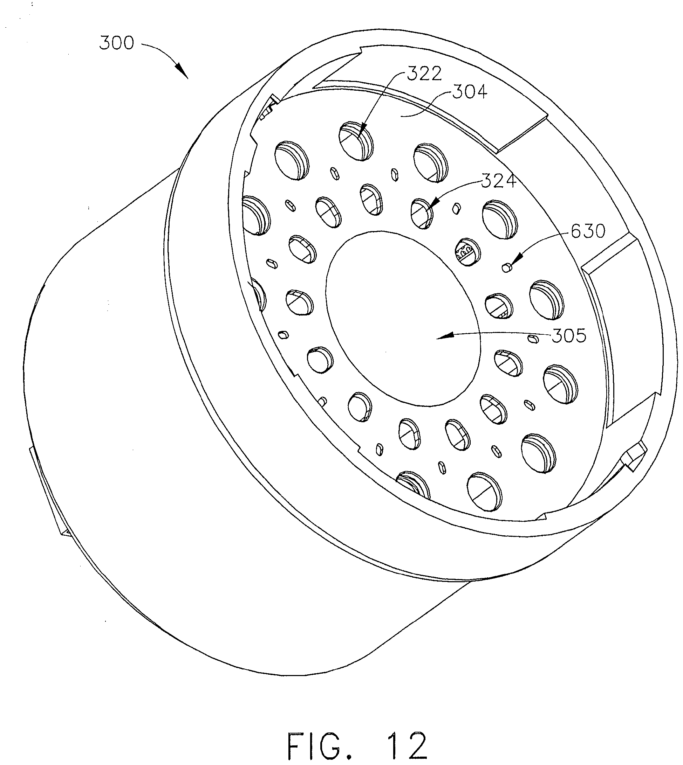

As shown in FIGS. 6A and 7A, pawl assembly (600) remains substantially stationary as cutter (600) is retracted from a distal position toward a proximal position. In the positioning shown in FIGS. 6A and 7A, pawls (604, 606) substantially hold housing (304) in place, thereby substantially maintaining the rotational position of housing (304) about the axis defined by shaft (352). In particular, pawls (604, 606) are received in respective recesses (630) of housing (304). Recesses (630) are shown in FIG.12 As cutter (200) reaches the proximal, fully retracted position as shown in FIG. 8A, pusher (240) engages block (602) and pushes block (602) proximally as cutter (200) finishes its proximal retraction movement. With pawls (604, 606) being disposed in respective recesses (630) of housing (304), this proximal movement of block (602) causes pawls (604, 606) to pivot and thereby rotate housing (304). The length of pawls (604, 606) is selected such that, when cutter (200) reaches the proximal, fully retracted position, the next chamber (316, 345) will be indexed to lumens (117, 204). In addition, as also seen in FIG. 8A, the chamfered alignment pin (612) is inserted into the upper aperture (322) of another chamber (316) as block (602) is moved proximally, thereby ensuring proper alignment of the proper chamber (316, 345) with lumens (117, 204). In the present example, alignment pin (602) is angularly positioned two chambers (316) over from the chamber (316) that is indexed to lumens (117, 204), though it should be understood that any other suitable positioning for alignment pin (602) may be used.

As cutter (200) begins to advance distally again, from the position shown in FIG. 8A to the position shown in FIG. 9A, pusher (240) leaves block (602). As pusher (240) leaves block (620), the distal bias provided by springs (610) pushes block (602) distally. As block (602) moves distally, alignment pin (602) leaves chamber (316), and pawls (604, 606) are dragged across the proximal face of housing (304) until they reach the next respective pair of recesses (630). Housing (304) is thus held stationary as cutter (200) continues its distal movement to the position shown in FIG. 6A, until cutter (200) is again retracted to the position shown in FIG. 8A to acquire another tissue sample. Thus, it should be understood that each time cutter (200) is actuated to sever a tissue sample, pawl assembly (600) will rotate housing (304) one chamber (316, 345) at a time to successively index chambers (316, 345) relative to lumens (117, 204), allowing separate tissue samples to be delivered to separate chambers (345).

In some versions, tissue sample holder (300) is configured an operable in accordance with the teachings of U.S. Pub. No. 2008/0221480, entitled "Biopsy Sample Storage," published September 11,2008. As another merely illustrative example, tissue sample holder (300) may be constructed and operable in accordance with the teachings of U.S. Pub. No. 2008/0214955. As yet another merely illustrative example, tissue sample holder (300) may be constructed and operable in accordance with the teachings of U.S. Pub. No. 2010/0160824, entitled "Biopsy Device with Discrete Tissue Chambers," published June 24, 2010. In some other versions, tissue sample holder (300) does not include a rotatable component. In some such versions, tissue sample holder (300) is constructed in accordance with the teachings of U.S. Provisional Patent App. No. 61/381,466, entitled "Biopsy Device Tissue Sample Holder with Removable Basket," filed September 10, 2010. Still other suitable ways in which tissue sample holder (300) may be constructed and operable will be apparent to those of ordinary skill in the art in view of the teachings herein.

### II. Exemplary Holster

As shown in FIGS. 1-2 and 13, holster (500) of the present example includes a top housing cover (502), side panels (504), and a housing base (506), which are fixedly secured together. Gears (521, 523) are exposed through top housing cover (502), and mesh with gears (121, 123) of probe (100) when probe (100) and holster (500) are coupled together. In particular, gears (521, 121) drive the mechanism that actuates cutter
(200); while gears (523, 123) are employed to rotate needle (110). Holster (500) also includes a firing rod (730) and fork (732), which couple with needle (110) and fire needle (110) distally as will be described in greater detail below.

### A. Exemplary Needle Rotation Mechanism

As noted above, rotation of gear (523) provides rotation of needle (110) relative to probe (100). In the present example, gear (523) is rotated by rotating knob (510). In particular, knob (510) is coupled with gear (523) by a series of gears (550, 552, 554, 556, 558, 560, 562) and shafts (570, 572, 574, 576, 578), such that rotation of knob (510) rotates gear (523). A second knob (510) extends from the other side of holster (700). By way of example only, such a needle rotation mechanism may be constructed in accordance with the teachings of U.S. Pub. No. 2008/0214955. As another merely illustrative example, a needle rotation mechanism may be constructed in accordance with the teachings of U.S. Pub. No. 2010/0160819. In some other versions, needle (110) is rotated by a motor. In still other versions, needle (110) is simply rotated by rotating thumbwheel (154). Various other suitable ways in which rotation of needle (110) may be provided will be apparent to those of ordinary skill in the art in view of the teachings herein. It should also be understood that some versions may provide no rotation of needle (110).

### B. Exemplary Needle Firing Mechanism

Holster (500) of the present example further includes a needle firing mechanism (400), which is operable to fire needle (110) from a loaded position to a fired position. By way of example only, such firing may be useful in instances where biopsy device (10) is mounted to a stereotactic table fixture or other fixture, with tip (112) adjacent to a patient's breast, such that needle firing mechanism (400) may be activated to drive needle (110) into the patient's breast. Needle firing mechanism (400) may be configured to drive needle (110) along any suitable range of motion, to drive tip (112) to any suitable distance relative to fixed components of probe (100). Needle firing mechanism (400) of the present example is loaded by pivoting arms (402) proximally. Needle firing
mechanism (400) is then fired by pressing firing button (404) while holding the associated arming trigger (406) in a distally rotated position.

In the present example, needle firing mechanism (400) is coupled with needle (110) via firing rod (732) and firing fork (732). Firing rod (732) and firing fork (734) are unitarily secured together. Firing fork (732) includes a pair of prongs (734) that receive hub member (150) of needle (110) therebeteween. Prongs (734) are positioned between annular flange (152) and thumbwheel (154), such that needle (110) will translate unitarily with firing rod (730) and fork (732). Prongs (734) nevertheless removably receive hub member (150), such that fork (732) may be readily secured to hub member (150) when probe (100) is coupled with holster (700); and such that hub member (150) may be readily removed from fork (732) when probe (100) is decoupled from holster (500). Prongs (734) are also configured to permit hub member (150) to rotate between prongs (734), such as when knob (510) is rotated to change the angular orientation of lateral aperture (114). Other suitable components, configurations, and relationships will be apparent to those of ordinary skill in the art in view of the teachings herein.

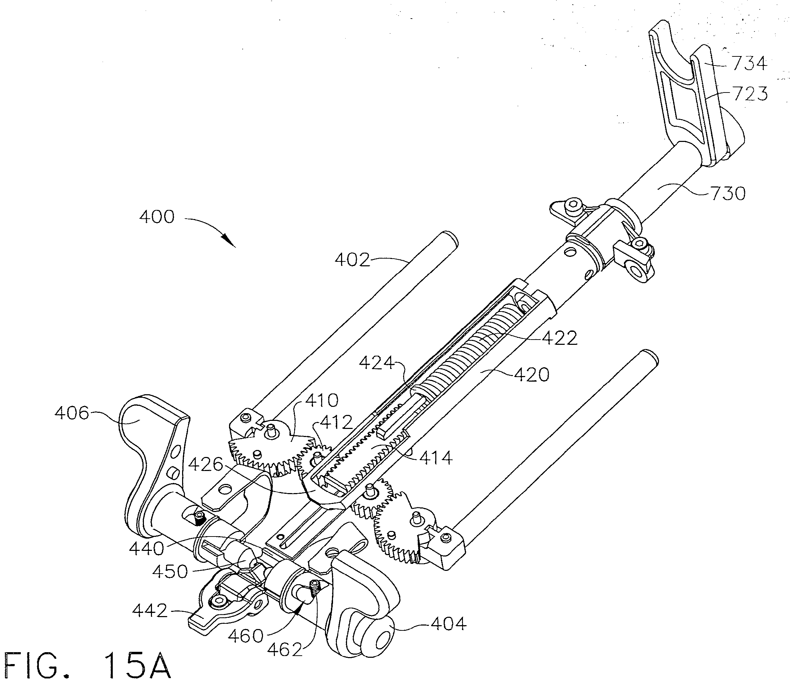

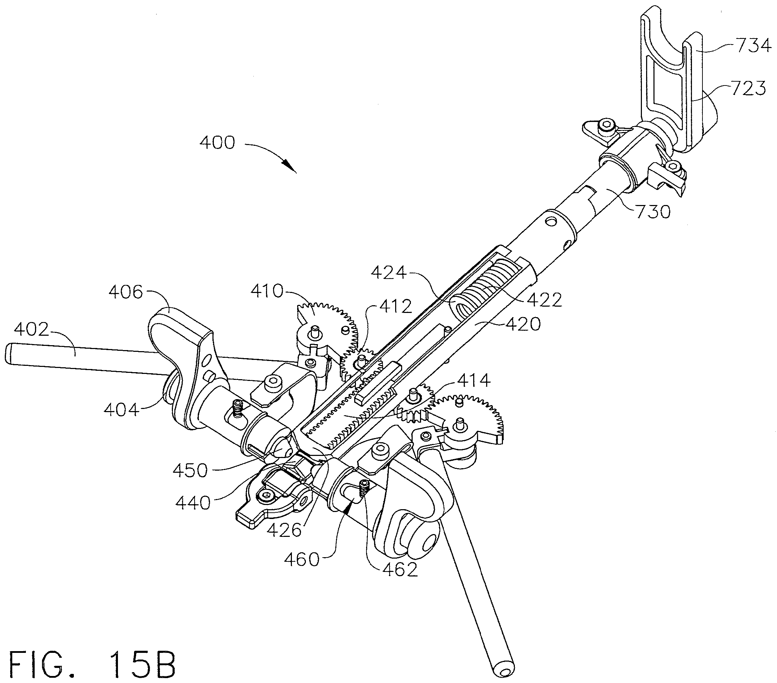

A gear (410) is fixedly secured to each arm (402), such that pivoting arm (402) relative to base (506) rotates gear (410) relative to base (506). Each gear (410) meshes with another respective gear (412). Both gears (412) mesh with opposite sides of a rack (414). Thus, it should be understood that pivoting arm (402) relative to base (506) will translate rack (414) longitudinally relative to base (506). Rack (414) is fixedly secured to a frame (420), which is fixedly secured to firing rod (730). A coil spring (422) resiliently biases frame (420) and thus firing rod (730) to a distal position as shown in FIG. 15B. The proximal end (424) of coil spring (422) is grounded by being fixedly secured relative to base (506). The proximal end of frame (420) includes a latch feature (426). Latch feature (426) is configured to selectively couple with a corresponding latch feature (440), to hold frame (420) and thus firing rod (730) at a proximal position as shown in FIG. 15A. Latch feature (440) is pivotally coupled to a clevis (442), which is fixedly secured relative to base (506). A resilient member (not shown) resiliently biases latch feature (440) to an upward position where latch feature (440) will engage with latch feature (426) when frame (420) reaches the proximal position shown in FIG. 15A. Latch features (426, 440) will stay engaged until latch feature (440) is pivoted downwardly.

Each firing button (404) includes an integral end cone (450) positioned near latch feature (440). As shown in FIG. 15A, each end cone (450) is configured to push latch feature (440) downwardly, and thus disengage latch feature (440) from latch feature (426), when firing button (404) is pressed in. However, arming trigger (406) is configured to prevent such pressing of firing button (404) unless arming trigger (406) is rotated to a distal rotational position as is also shown in FIG. 15A. In particular, arming trigger (406) defines an L-shaped slot (460). A pin (462) that is fixedly secured to each firing button (404) is disposed in a corresponding L-shaped slot (460). The configurations and engagement between slots (460) and pins (462) prevents either firing button (404) from being pressed sufficiently inwardly unless the corresponding arming trigger (406) is rotated to a distal rotational position. A resilient member (not shown) resiliently biases each button (404) to an outward position. Another resilient member resiliently biases each trigger (406) to an upright, non-rotated position.

In an exemplary use of needle firing mechanism (400), firing rod (730) is initially at a distal position as shown in FIG. 15A. Then arms (402) are pivoted proximally to the position shown in FIG. 15B. It should be understood that a user may elect to push or pull on just one arm (402) or both arms (402). As arms (402) are pivoted proximally, frame (420) and firing rod (730) are also pulled proximally, causing spring (422) to compress. Once frame (420) reaches the proximal position, latch features (426, 440) engage to hold frame (420) and firing rod (730) in the proximal position, resisting the distal bias of compressed spring (422). With needle (110) being secured to fork (732), needle (110) is thus in a cocked configuration. In order to fire needle (110) distally, a user rotates an arming trigger (406) distally and presses the associated button (404) inwardly. This is shown in the left button (404) and trigger (406) in FIG. 15A. With button (404) pressed inwardly, the associated end cone (450) cammingly causes latch feature (440) to pivot downwardly, such that latch feature (440) disengages latch feature (426). With latch features (426, 440) disengaged, spring (422) immediately and forcefully decompresses, rapidly pushing distally on firing bar (730) and fork (732) to fire needle (110) into the patient's breast. With needle (110) having been fired into the breast, the user may then activate the cutter actuation mechanism to acquire one or more biopsy samples from the patient's breast.

As another merely illustrative alternative, needle firing mechanism (400) may be constructed in accordance with at least some of the teachings of U.S. Pub. No. 2008/0214955. As yet another merely illustrative alternative, needle firing mechanism (400) may be constructed in accordance with at least some of the teachings of U.S. Non-Provisional Patent App. No. 13/086,567, entitled "Biopsy Device with Motorized Needle Firing," filed April 14, 2011. Various other suitable ways in which needle firing mechanism (400) may be constructed and operable will be apparent to those of ordinary skill in the art in view of the teachings herein. In some other versions, needle firing mechanism (400) is omitted entirely. For instance, biopsy device (10) may be constructed such that needle (110) simply does not fire relative to probe (100) and/or relative to holster (500)

### III. Exemplary Tissue Sensing Components

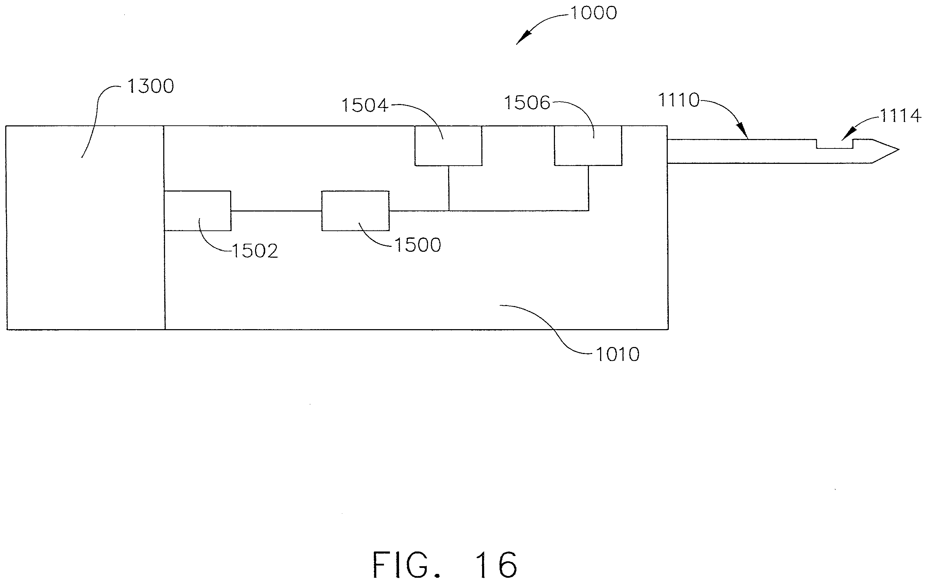

In some settings, it may be desirable to know when a sufficient tissue sample has been captured by a biopsy device during a sampling process. For instance, it may be desirable to sense whether a tissue sample of sufficient size has been captured. As another merely illustrative example, it may be desirable to sense whether a tissue sample has successfully traveled to a tissue sample holder in a biopsy device. With such information, the user may be provided with an alert and/or the operation of biopsy device may be influenced. Examples of how such information may be acquired and what may be done with it are described below with reference to FIGS. 16-17, while other examples will be apparent to those of ordinary skill in the art in view of the teachings herein.

FIG. 16 depicts a biopsy device (1000) that includes a body (1010), a needle (1110) extending distally from body (1010) and a tissue sample holder (1300) coupled with body (1010). Biopsy device (1000) is operable to capture tissue samples through a lateral aperture (1114) formed in needle (1110) and deposit those tissue samples in tissue sample holder (1300). By way of example only, biopsy device (1000) may be
constructed and operable in accordance with any variation biopsy device (10) described above. Alternatively, biopsy device (1000) may be constructed and operable in accordance with at least some of the teachings of any of the reference cited herein, including combinations of teachings from different references cited herein. Alternatively, biopsy device (1000) may have any other suitable configuration and operability.

Biopsy device (1000) of the present example includes a processing module (1500), a tissue sensor (1502), an audible indicator (1504), and a visual indicator (1506). Tissue sensor (1502), audible indicator (1504), and visual indicator (1506) are all in communication with processing module (1500). Processing module (1500) may comprise a printed circuit board, one or more microprocessors, and/or various other types of components as will be apparent to those of ordinary skill in the art in view of the teachings herein.

Tissue sensor (1502) is shown as being positioned adjacent to tissue sample holder (1300), though it should be understood that tissue sensor (1502) may be positioned at any other suitable location. By way of example only, tissue sensor (1502) may be located within tissue sample holder (1300), in or adjacent to a lumen through which tissue samples are communicated to reach tissue sample holder (1300), etc. It should also be understood that biopsy device (1000) may have more than one tissue sensor (1502). Tissue sensor (1502) may take a variety of forms. For instance, tissue sensor (1502) may comprise an ultrasonic sensor that is configured to sense tissue samples as a disturbance in an ultrasonic field. Tissue sensor (1502) may alternatively comprise a laser sensor configured to detect tissue samples as a break in a laser beam projected across a tissue sample path. Tissue sensor (1502) may alternatively comprise a capacitive sensor, with processing module (1500) being configured to distinguish between tissue samples and fluids that are sensed by capacitive sensor. Tissue sensor (1502) may alternatively comprise a Doppler sensor, a strain gauge, an optical sensor, or a proximity sensor. As yet another merely illustrative alternative, tissue sensor (1502) may comprise a vacuum sensor that senses the acquisition of tissue samples based on variations of vacuum strength. In addition or in the alternative, such a vacuum sensor may be configured and/or used in accordance with any of the teachings in U.S. Pub. No. 2009/0171243, entitled "Vacuum Sensor and Pressure Pump for Tetherless Biopsy Device," published July 2, 2009. As still another merely illustrative example, tissue sensor (1502) may comprise a mechanical member positioned in the path of tissue, such that the mechanical member will move as tissue contacts the mechanical member during proximal transport of the tissue sample to tissue sample holder (1300), thereby causing a sensor or momentary switch to send a signal that tissue had been transported. Still other suitable forms that tissue sensor (1502) may take will be apparent to those of ordinary skill in the art in view of the teachings herein.

In some versions, tissue sensor (1502) is configured to simply sense whether a tissue sample has been captured. In addition, tissue sensor (1502) may be configured to sense qualities of a captured tissue sample, such as length, mass, color, etc. Various suitable ways in which one or more tissue sensors (1502) may sense qualities of a captured tissue sample such as length, mass, color, etc. will be apparent to those of ordinary skill in the art in view of the teachings herein.

Audible indicator (1504) of the present example comprises a speaker or other sound emitting device that is operable to emit sounds that are audible to a user. Processing module (1500) is programmed to drive audible indicator (1504) based on information acquired from tissue sensor (1502). For instance, audible indicator (1504) may beep or chime, etc., when tissue sensor (1502) senses a captured tissue sample, such as when a captured tissue sample has reached tissue sample holder (1300). It should also be understood that control module (1500) may be programmed to drive audible indicator (1504) to produce different sounds based on different conditions detected by tissue sensor (1502). For instance, if information from tissue sensor (1502) and/or other components of biopsy device (1000) indicate that tissue has become jammed in biopsy device (1000), audible indicator (1504) may provide an audible alert that is different from one provided when a tissue sample successfully reaches tissue sample holder (1300). Similarly, in instances where one or more tissue sensors (1502) are operable to sense qualities of a captured tissue sample such as length, mass, color, etc., such qualities
may be represented by different sounds emitted by audible indicator (1504). The sound from audible indicator (1504) may be varied in numerous ways, including but not limited to tone, pitch, timbre, volume, pattern, rhythm, melody, etc. Other suitable ways in which audible indicator (1504) may be used/provided will be apparent to those of ordinary skill in the art in view of the teachings herein. Alternatively, audible indicator (1504) may simply be omitted if desired.

Visual indicator (1506) of the present example comprises an LED. Alternatively, visual indicator (1506) may comprise a plurality of LEDs, a graphical display, and/or any other suitable component/feature (or combination thereof) that is/are operable to provide some form of visual indication to a user. In some versions, tissue sample holder (1300) is selectively illuminated by visual indicator (1506) (e.g., flashing light and/or changing light color, etc.) to indicate capture of a tissue sample. Processing module (1500) is programmed to drive visual indicator (1506) based on information acquired from tissue sensor (1502), similar to the way in which audible indicator (1504) is driven as described above. For instance, visual indicator (1506) may illuminate when tissue sensor (1502) senses a captured tissue sample, such as when a captured tissue sample has reached tissue sample holder (1300). It should also be understood that control module (1500) may be programmed to drive visual indicator (1506) to produce different sounds based on different conditions detected by tissue sensor (1502). For instance, if information from tissue sensor (1502) and/or other components of biopsy device (1000) indicate that tissue has become jammed in biopsy device (1000), visual indicator (1506) may provide a visual alert that is different from one provided when a tissue sample successfully reaches tissue sample holder (1300). Similarly, in instances where one or more tissue sensors (1502) are operable to sense qualities of a captured tissue sample such as length, mass, color, etc., such qualities may be represented by different visual cues provided by visual indicator (1506). The visual indication from visual indicator (1506) may be varied in numerous ways, including but not limited to color, graphics, pattern, rhythm, etc. Visual indicator (1506) may also provide a count of the number of tissue samples detected by tissue sensor (1502) during operation of biopsy device (1000). Other suitable ways in which visual indicator (1506) may be used/provided will be apparent to those of ordinary skill in the art in view of the teachings herein. Alternatively, visual indicator (1506) may simply be omitted if desired.

It should also be understood that processing module (1500) may affect operation of biopsy device (1000) based at least in part on information from tissue sensor (1502), in addition to or in lieu of providing feedback to the user via audible indicator (1504) and/or visual indicator (1506). For instance, in instances where processing module (1500) is operable to influence operation of a cutter actuation mechanism, processing module (1500) may be configured to provide automatic repeated actuation of a cutter until a satisfactory tissue sample is detected by tissue sensor (1502). In addition or in the alternative, processing module (1500) may be configured to control the level of vacuum, such as by strengthening the vacuum for a second cutting cycle when tissue sensor (1502) fails to detect a sufficient tissue sample after a first cutting cycle. Still other ways in which processing module (1500) may influence operation of biopsy device (1000) based at least in part on information from tissue sensor (1502) will be apparent to those of ordinary skill in the art in view of the teachings herein.

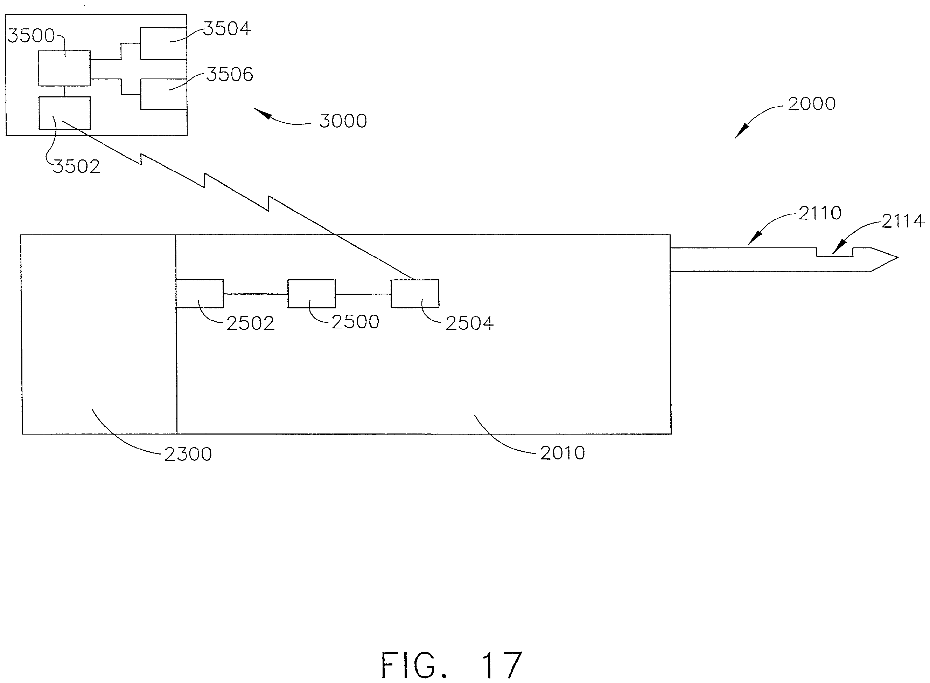

FIG. 17 shows another exemplary biopsy device (2000) that includes a body (2010), a needle (2110) extending distally from body (2010) and a tissue sample holder (2300) coupled with body (2010). Biopsy device (2000) is operable to capture tissue samples through a lateral aperture (2114) formed in needle (2110) and deposit those tissue samples in tissue sample holder (2300). By way of example only, biopsy device (2000) may be constructed and operable in accordance with any variation biopsy device (10, 1000) described above. Alternatively, biopsy device (2000) may be constructed and operable in accordance with at least some of the teachings of any of the reference cited herein, including combinations of teachings from different references cited herein. Alternatively, biopsy device (2000) may have any other suitable configuration and operability.

Biopsy device (2000) of the present example includes a processing module (2500), a tissue sensor (2502), and a transmitter (2504). Tissue sensor (2502) and transmitter (2504) are in communication with processing module (2500). Processing module (2500) may comprise a printed circuit board, one or more microprocessors, and/or various other types of components as will be apparent to those of ordinary skill in the art in view of the teachings herein. Tissue sensor (2502) is shown as being positioned adjacent to tissue sample holder (2300), though it should be understood that tissue sensor (2502) may be positioned at any other suitable location. By way of example only, tissue sensor (2502) may be located within tissue sample holder (2300), in or adjacent to a lumen through which tissue samples are communicated to reach tissue sample holder (2300), etc. It should also be understood that biopsy device (1000) may have more than one tissue sensor (2502). Tissue sensor (2502) may take a variety of forms, including but not limited to any of the forms discussed above with reference to tissue sensor (1502). Still other suitable forms that tissue sensor (2502) may take will be apparent to those of ordinary skill in the art in view of the teachings herein. As with tissue sensor (1502) described above, tissue sensor (2502) may also be configured to sense qualities of a captured tissue sample, such as length, mass, color, etc.

Transmitter (2504) of the present example is operable to provide wireless communication with a remote unit (3000). Remote unit (3000) of this example includes a processing module (3500), a receiver (3502), an audible indicator (3504), and a visual indicator (3506). Receiver (3502), audible indicator (3502), and visual indicator (3506) are all in communication with processing module (3500). Processing module (3500) may comprise a printed circuit board, one or more microprocessors, and/or various other types of components as will be apparent to those of ordinary skill in the art in view of the teachings herein. Receiver (3502) is operable to receive wireless communications from transmitter (2504), including but not necessarily limited to information from tissue sensor (2502). Transmitter (2504) and receiver (3502) may use any suitable mode of wireless communication, including but not limited to RF communication using any suitable protocol (e.g., Bluetooth, Zigbee, etc.). It should also be understood that transmitter (2504) and receiver (3502) may provide bi-directional communication, such that transmitter (2504) and receiver (3502) are each capable of acting as a transceiver. Furthermore, it should be understood that biopsy device (2000) and remote unit (3000) may be in communication via one or more wires, in addition to or in lieu of being in communication wirelessly.

Audible indicator (3504) of the present example comprises a speaker or other sound emitting device that is operable to emit sounds that are audible to a user. Processing module (3500) is programmed to drive audible indicator (3504) based on information acquired from tissue sensor (2502). For instance, processing module (3500) may drive audible indicator (3504) in any suitable fashion as discussed above with respect to audible indicator (1504). Other suitable ways in which audible indicator (3504) may be used/provided will be apparent to those of ordinary skill in the art in view of the teachings herein. Alternatively, audible indicator (3504) may simply be omitted if desired.

Visual indicator (3506) of the present example comprises an LED. Alternatively, visual indicator (3506) may comprise a plurality of LEDs, a graphical display, and/or any other suitable component/feature (or combination thereof) that is/are operable to provide some form of visual indication to a user. Processing module (3500) is programmed to drive visual indicator (3506) based on information acquired from tissue sensor (2502), similar to the way in which visual indicator (3504) is driven as described above. In some versions, visual indicator (3506) provides a visual indication to indicate when tissue has been successfully transported and/or when tissue has not been successfully transported. Similarly, visual indicator (3506) may provide an indication to the user that the user needs to continue attempting to capture biopsy samples because tissue did not successfully transport during previous attempts.

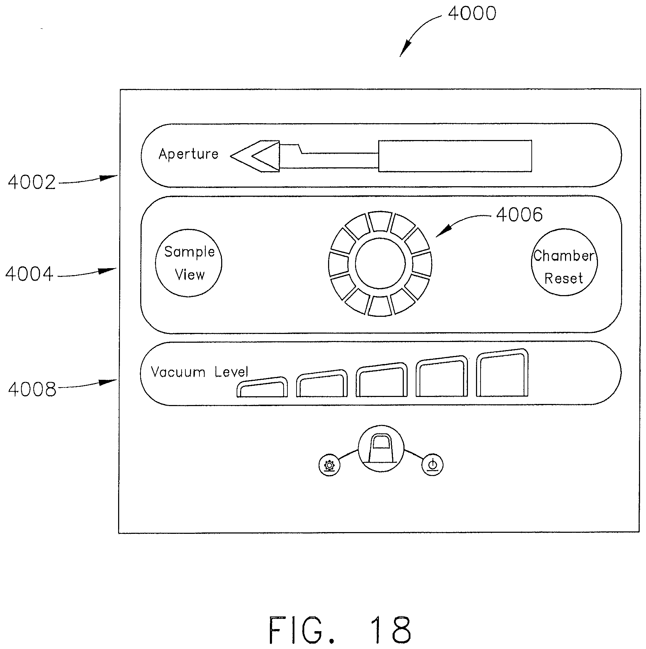

One merely illustrative example of how visual indicator (3506) may be provided is shown in FIG. 18, which shows an exemplary user interface (4000). User interface (4000) of this example includes a cutter position indicator (4002), a tissue chamber occupancy indicator (4004), and a vacuum level indicator (4008). Cutter position indicator (4002) shows the position of a cutter relative to side aperture (2114) of needle (2110). Tissue chamber occupancy indicator (4004) shows which chambers in a multi-chamber tissue sample holder (2300) are occupied by tissue samples. In particular, indicator (4004) includes discrete representations (4006) of each tissue sample chamber of tissue sample holder (2300). In some versions, when a chamber of tissue sample holder (2300) receives a tissue sample, the representation (4006) for that chamber illuminates, changes color, or otherwise visually indicates the occupancy by a tissue sample. In addition or in the alternative, indicator (4004) may collectively rotate all representations (4006) each time a chamber of tissue sample holder (2300) receives a tissue sample. Such rotation may mimic rotation of a manifold or other component within tissue sample holder (2300) (e.g., the portion that successively indexes chambers relative to the cutter). For instance, indicator (4004) may collectively rotate all representations (4006) each time a chamber of tissue sample holder (2300) receives a tissue sample to keep the representation (4006) of the next adjacent empty chamber located at the uppermost rotational position (e.g., the 12 o'clock position). Indicator (4004) may thus collectively rotate all representations (4006) clockwise or counter-clockwise, one chamber representation (4006) at a time, each time a chamber of tissue sample holder (2300) receives a tissue sample, in synchronization with actual movement of chambers in tissue sample holder (2300).

In addition or in the alternative, indicator (4004) may illuminate one or more empty chamber representations (4006). For instance, indicator (4004) may illuminate the representation (4006) of the next adjacent empty chamber each time a chamber of tissue sample holder (2300) receives a tissue sample, thereby indicating that the chamber represented by the illuminated representation (4006) is the "active" chamber (i.e., that the chamber represented by the illuminated representation (4006) is the chamber that is indexed relative to the cutter for receipt of the next tissue sample). As yet another merely illustrative example, some versions of biopsy device (2000) may include a sample viewing mode whereby a component of tissue sample holder (2300) rotates each time a tissue sample is acquired, to present the most recently occupied tissue sample chamber to the user. For instance, the most recently occupied tissue sample chamber may be rotated to a 9 o'clock rotational position or a 3 o'clock rotational position, to facilitate viewing from the side of biopsy device (2000). Such positioning of the most recently occupied tissue sample chamber may be temporary, such that after presenting the most recently occupied tissue sample chamber to the user for a predetermined time period (e.g., one or two seconds, etc.), the component of tissue sample holder (2300) rotates again to index the adjacent empty chamber relative to the cutter. Examples of such a sample viewing mode and associated operation are disclosed in U.S. Pub. No. 2008/0214955. In versions where such a mode is used, indicator (4004) may track such movement of the chambers by collectively rotating representations (4006) in synchronization with rotational movement of the actual chambers of tissue sample holder (2300).

Indications provided through indicator (4004) may be based at least in part on information from tissue sensor (2502). In addition or in the alternative, such indications may be based at least in part on other information, including but not limited to information from a sensor that tracks motion of the cutter, the rotatable housing of tissue sample holder (2300), or some other component of biopsy device (2000). It should therefore be understood that user interface (4000) could be used with virtually any biopsy device, including those lacking a tissue sensor (2502), such as any biopsy device that is described herein and/or any biopsy device that is described in any reference cited herein that includes disclosure of a multi-chamber tissue sample holder.

Other suitable ways in which visual indicator (3506) may be used/provided will be apparent to those of ordinary skill in the art in view of the teachings herein. Alternatively, visual indicator (3506) may simply be omitted if desired.

In some versions, remote unit (3000) is a dedicated device constructed specifically to provide audio and/or visual feedback to a user based on information from tissue sensor (3502). In some other versions, remote unit (3000) is also configured to perform other functions that are not necessarily based on information from tissue sensor (3502). For instance, remote unit (3000) may comprise a display screen in an ultrasound imaging system. In some such versions, a user may be able to continue watching a real time image of a biopsy site under ultrasound while acquiring tissue samples at the biopsy site with biopsy device (3000), and may be able to receive indications from one or both indicators (3504, 3506) through the ultrasound imaging system without having to look away from the display screen of the ultrasound imaging system. As another merely illustrative example, remote unit (3000) may be provided as a pod or box that mounts on, sits on, is secured to, or is otherwise positioned near a display screen of an ultrasound imaging system, again permitting a user to receive indications from one or both indicators (3504, 3506) through the pod or box without having to look away from the display screen of the ultrasound imaging system. As another example, remote unit (3000) may be integrated into a vacuum control module, such as a vacuum control module as described in U.S. Pub. No. 2008/0214955. Still other suitable ways in which remote unit (3000) may be provided will be apparent to those of ordinary skill in the art in view of the teachings herein.

Embodiments of the present invention have application in conventional endoscopic and open surgical instrumentation as well as application in robotic-assisted surgery.

Embodiments of the devices disclosed herein can be designed to be disposed of after a single use, or they can be designed to be used multiple times. Embodiments may, in either or both cases, be reconditioned for reuse after at least one use. Reconditioning may include any combination of the steps of disassembly of the device, followed by cleaning or replacement of particular pieces, and subsequent reassembly. In particular, embodiments of the device may be disassembled, and any number of the particular pieces or parts of the device may be selectively replaced or removed in any combination. Upon
cleaning and/or replacement of particular parts, embodiments of the device may be reassembled for subsequent use either at a reconditioning facility, or by a surgical team immediately prior to a surgical procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, embodiments described herein may be processed before surgery. First, a new or used instrument may be obtained and if necessary cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, or high-energy electrons. The radiation may kill bacteria on the instrument and in the container. The sterilized instrument may then be stored in the sterile container. The sealed container may keep the instrument sterile until it is opened in a medical facility. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A biopsy system, comprising:
(a) a needle (2110);
(b) a cutter (200) movable relative to the needle to sever a tissue sample; and
(c) a tissue sample holder (2300) in communication with the cutter, wherein the tissue sample holder comprises a plurality of chambers (345) and wherein a portion of the tissue sample holder is configured to rotate to successively index the chambers relative to the cutter;
**characterised in that** the system further comprises:
(d) a processing module (3500); and
(e) a graphical user interface (4000) in communication with the processing module, wherein the graphical user interface is operable to display an indicator (4004) comprising a visual representation of each of the chambers (4006),
wherein:
the processing module is operable to drive the indicator to indicate which chambers are occupied by severed tissue samples through the visual representations of the chambers by rotating the indicator in response to receipt of a tissue sample in the corresponding chamber, thereby positioning the visual representation of an adjacent empty chamber at a predetermined location; and
the processing module is operable to drive the rotation of the indicator to collectively rotate all representations clockwise or counter-clockwise each time a chamber of the tissue sample holder receives a tissue sample in synchronization with actual movement of chambers in the tissue sample holder.

2. The biopsy system of claim 1, further comprising a biopsy instrument (2000), wherein the needle, the cutter, and the tissue sample holder are integrated into the biopsy instrument, the system further comprising a remote unit separate from the biopsy instrument, wherein the graphical user interface is integrated into the remote unit.

3. The biopsy system of claim 1, further comprising a tissue sensor (2502).

4. The biopsy system of claim 3, wherein the visual representation of the chambers is based at least in part on information from the tissue sensor.

5. The biopsy system of claim 3, wherein the tissue sensor comprises a sensor selected from the group consisting of an ultrasonic sensor, a capacitive sensor, a laser sensor, an optical sensor, and a Doppler sensor.

6. The biopsy system of claim 1, wherein the biopsy system is operable to indicate occupation of a chamber by illuminating a visual representation of a chamber.

7. The biopsy system of claim 1, further comprising a tissue sensor (2502) in communication with the processing module, wherein the tissue sensor is configured to sense a tissue sample severed by the cutter.

## Patentansprüche

1. Biopsiesystem, umfassend:
(a) eine Nadel (2110);
(b) eine Schneideeinrichtung (200), die zum Durchtrennen einer Gewebeprobe bezüglich der Nadel beweglich ist; und
(c) einen Gewebeprobenhalter (2300) in Verbindung mit der Schneideeinrichtung, wobei der Gewebeprobenhalter eine Vielzahl von Kammern (345) umfasst und wobei ein Abschnitt des Gewebeprobenhalters dazu ausgelegt ist, sich zu drehen, um die Kammern bezüglich der Schneideeinrichtung sukzessive zu indexieren;
**dadurch gekennzeichnet, dass** das System ferner Folgendes umfasst:
(d) ein Verarbeitungsmodul (3500); und
(e) eine graphische Benutzeroberfläche (4000) in Verbindung mit dem Verarbeitungsmodul, wobei die graphische Benutzeroberfläche zur Anzeige eines Indikators (4004) betätigbar ist, der eine visuelle Darstellung jeder Kammer (4006) umfasst,
wobei:
das Verarbeitungsmodul zum Antreiben des Indikators betätigbar ist, um durch visuelle Darstellungen der Kammern anzuzeigen, welche Kammern von durchtrennten Gewebeproben besetzt sind, indem der Indikator als Reaktion auf die Aufnahme einer Gewebeprobe in der entsprechenden Kammer gedreht wird, wodurch die visuelle Darstellung einer benachbarten leeren Kammer an einer vorbestimmten Stelle platziert wird; und
das Verarbeitungsmodul zum Antreiben der Drehung des Indikators betätigbar ist, um jedes Mal, wenn eine Kammer des Gewebeprobenhalters eine Gewebeprobe aufnimmt, in Synchronisation mit der tatsächlichen Bewegung der Kammern im Gewebeprobenhalter alle Darstellungen kollektiv im Uhrzeigersinn oder gegen den Uhrzeigersinn zu drehen.

2. Biopsiesystem nach Anspruch 1, ferner umfassend ein Biopsieinstrument (2000), wobei die Nadel, die Schneideeinrichtung und der Gewebeprobenhalter in dem Biopsieinstrument integriert sind, wobei das System ferner eine Fernbedienungseinheit umfasst, die von dem Biopsieinstrument getrennt ist, wobei die graphische Benutzeroberfläche in der Fernbedienungseinheit integriert ist.

3. Biopsiesystem nach Anspruch 1, ferner einen Gewebesensor (2502) umfassend.

4. Biopsiesystem nach Anspruch 3, wobei die visuelle Darstellung der Kammern wenigstens teilweise aus Informationen vom Gewebesensor basiert.

5. Biopsiesystem nach Anspruch 3, wobei der Gewebesensor einen Sensor umfasst, der aus der aus einem Ultraschallsensor, einem kapazitiven Sensor, einem Lasersensor, einem optischen Sensor und einem Doppler-Sensor bestehenden Gruppe ausgewählt ist.

6. Biopsiesystem nach Anspruch 1, wobei das Biopsiesystem zur Anzeige der Besetzung einer Kammer durch Beleuchtung einer visuellen Darstellung einer Kammer betätigbar ist.

7. Biopsiesystem nach Anspruch 1, ferner umfassend einen Gewebesensor (2502) in Verbindung mit dem Verarbeitungsmodul, wobei der Gewebesensor dazu ausgelegt ist, eine von der Schneideeinrichtung durchtrennte Gewebeprobe zu erkennen.

## Revendications

1. System de biopsie, comprenant :
(a) une aiguille (2110) ;
(b) un organe de coupe (200) mobile par rapport à l'aiguille pour trancher un échantillon de tissu ; et
(c) un porte-échantillon de tissu (2300) en communication avec l'organe de coupe, le porte-échantillon de tissu comprenant une pluralité de chambres (345) et une portion du porte-échantillon de tissu étant configurée pour tourner pour indexer successivement les chambres par rapport à l'organe de coupe ;
**caractérisé en ce que** le système comprend en outre :
(d) un module de traitement (3500) ; et
(e) une interface utilisateur graphique (4000) en communication avec le module de traitement, l'interface utilisateur graphique étant opérationnelle pour afficher un indicateur (4004) comprenant une représentation visuelle de chacune des chambres (4006),
dans lequel :
le module de traitement est opérationnel pour entraîner l'indicateur à indiquer les chambres qui sont occupées par des échantillons de tissu tranchés par le biais des représentations visuelles des chambres en faisant tourner l'indicateur en réponse à une réception d'un échantillon de tissu dans la chambre correspondante, positionnant ainsi la représentation visuelle d'une chambre vide adjacente à un emplacement prédéterminé ; et
le module de traitement est opérationnel pour entraîner la rotation de l'indicateur pour faire tourner collectivement toutes les représentations dans le sens horaire ou antihoraire à chaque fois qu'une chambre du porte-échantillon de tissu reçoit un échantillon de tissu en synchronisation avec le mouvement effectif des chambres dans le porte-échantillon de tissu.

2. Système de biopsie selon la revendication 1, comprenant en outre un instrument de biopsie (2000), dans lequel l'aiguille, l'organe de coupe et le porte-échantillon de tissu sont intégrés dans l'instrument de biopsie, le système comprenant en outre une unité à distance séparée de l'instrument de biopsie, dans lequel l'interface utilisateur graphique est intégrée dans l'unité à distance.

3. Système de biopsie selon la revendication 1, comprenant en outre un capteur de tissu (2502).

4. Système de biopsie selon la revendication 3, dans lequel la représentation visuelle des chambres est basée au moins en partie sur des informations issues du capteur de tissu.

5. Système de biopsie selon la revendication 3, dans lequel le capteur de tissu comprend un capteur choisi dans le groupe consistant en un capteur ultrasonore, un capteur capacitif, un capteur laser, un capteur optique et un capteur Doppler.

6. Système de biopsie selon la revendication 1, dans lequel le système de biopsie est opérationnel pour indiquer l'occupation d'une chambre par illumination d'une représentation visuelle d'une chambre.

7. Système de biopsie selon la revendication 1, comprenant en outre un capteur de tissu (2502) en communication avec le module de traitement, dans lequel le capteur de tissu est configuré pour détecter un échantillon de tissu tranché par l'organe de coupe.
